# EUROPEAN PATENT SPECIFICATION

(11) **EP 1 723 246 B1**
(45) Date of publication and mention of the grant of the patent: **09.09.2009**
(21) Application number: 04803271.8
(22) Date of filing: 25.11.2004
(51) Int. Cl.: C12N 15/866, C12N 15/10, A01K 67/027, A61K 31/7088

(54) **NEW EXPRESSION TOOLS FOR MULTIPROTEIN APPLICATIONS**
NEUE EXPRESSIONSWERKZEUGE FÜR MULTIPROTEINANWENDUNGEN
NOUVEAUX OUTILS D'EXPRESSION POUR APPLICATIONS MULTIPROTEIQUES

(30) Priority: 09.03.2004 US 552072 P
(43) Date of publication of application: 22.11.2006
(73) Proprietor: Eidgenössische Technische Hochschule Zürich, 8092 Zürich (CH)
(72) Inventor: BERGER, Imre, CH-8046 Zurich (CH); FITZGERALD, Daniel, J., CH-8049 Zurich (CH); RICHMOND, Timothy, CH-8049 Zurich (CH)
(74) Representative: Kasche, André
(86) International application number: PCT/EP2004/013381
(87) International publication number: WO 2005/085456

(56) References cited:
- DATABASE INVITROGEN ONLINE pcDNA3.1 (-) Neo data sheet and restriction map XP002323547 -& KONE BRUCE C ET AL: "A novel N-terminal splice variant of the rat H+-K+-ATPase alpha2 subunit" 30 January 1998 (1998-01-30), JOURNAL OF BIOLOGICAL CHEMISTRY, VOL. 273, NR. 5, PAGE(S) 2543-2552 , XP002323545 ISSN: 0021-9258
- DATABASE INVITROGEN ONLINE pREP7/CAT data sheet and restriction map XP002323548 -& BOYLE J S ET AL: "ENHANCED RESPONSES TO A DNA VACCINE ENCODING A FUSION ANTIGEN THAT IS DIRECTED TO SITES OF IMMUNE INDUCTION" NATURE, MACMILLAN JOURNALS LTD. LONDON, GB, vol. 392, 1998, pages 408-411, XP000914533 ISSN: 0028-0836
- DATABASE INVITROGEN ONLINE pFastBac Dual data sheet and restriction map XP002323549 -& PHILIPPS BJORN ET AL: "Baculovirus expression system for magnetic sorting of infected cells and enhanced titer determination." BIOTECHNIQUES, vol. 36, no. 1, January 2004 (2004-01), pages 80-83, XP009045799 ISSN: 0736-6205
- LIN LI ET AL: "Efficient linking and transfer of multiple genes by a multigene assembly and transformation vector system." PROCEEDINGS OF THE NATIONAL ACADEMY OF SCIENCES OF THE UNITED STATES OF AMERICA, vol. 100, no. 10, 13 May 2003 (2003-05-13), pages 5962-5967, XP002323544 ISSN: 0027-8424
- HÜSER ANDREAS ET AL: "Baculovirus vectors: novel mammalian cell gene-delivery vehicles and their applications." 2003, AMERICAN JOURNAL OF PHARMACOGENOMICS : GENOMICS-RELATED RESEARCH IN DRUG DEVELOPMENT AND CLINICAL PRACTICE. 2003, VOL. 3, NR. 1, PAGE(S) 53 - 63 , XP009045490 ISSN: 1175-2203 page 59, left-hand column, paragraph 3 - page 61, last paragraph table I
- DE FELIPE P: "Polycistronic viral vectors" September 2002 (2002-09), CURRENT GENE THERAPY, VOL. 2, NR. 3, PAGE(S) 355-378 , XP001205951 ISSN: 1566-5232 cited in the application the whole document
- BERGER IMRE ET AL: "Baculovirus expression system for heterologous multiprotein complexes." December 2004 (2004-12), NATURE BIOTECHNOLOGY. DEC 2004, VOL. 22, NR. 12, PAGE(S) 1583 - 1587 , XP002323546 ISSN: 1087-0156 the whole document

## Description

The present invention relates to polynucleotides for multigene applications comprising a novel functional arrangement, as well as vectors, host cells, and recombinant animals comprising said polynucleotides.

In addition, the present invention is directed to methods for generating multigene expression cassettes, methods for producing multiprotein complexes *in vitro* and *in vivo,* and methods for producing a vaccine.

Furthermore, the present invention encompasses methods for screening protein complex interactions or modifications of proteins, and methods for the *in vitro* or *in vivo* screening of candidate compounds capable of protein complex interactions or modifications of proteins or capable of inhibiting protein complex interactions or inhibiting modifications of proteins.

Also, the present invention relates to the use of the polynucleotides, vectors, host cells or recombinant animals of the invention for (i) preparing a medicament for gene therapy, for (ii) the recombinant production of multiprotein complexes, (iii) for producing a vaccine, or (iv) for screening compounds of interest.

Last but not least, the invention is directed to a kit of parts comprising at least a polynucleotide, a vector, and/or a host cell according to the invention.

### Background of the invention

The identification of novel multiprotein complexes in eukaryotic cells has accelerated considerably, in particular due to the advent of genome-wide analysis of protein-protein interactions, the introduction of powerful multiple-affinity protein purification methods and the development of ultrasensitive analytical techniques. Based on extensive two-hybrid searches, the number of interaction partners for a given protein was estimated for baker's yeast to range on average from five to eight (Schwikowski et al. A network of protein-protein interactions in yeast. Nat. Biotechnol. 18, 1257-1261 (2000); Bader et al. Analyzing protein-protein interaction data obtained from different sources. Nat. Biotechnol. 20, 991-997 (2002); Von Mering et al. Comparative assessment of large-scale data sets of protein-protein interactions. Nature 417, 399-403 (2002); Grigoriev, A. On the number of protein-protein interactions in the yeast proteome. Nucleic Acids Res. 31, 4157-4161 (2003)). The concept of the cell as a collection of multicomponent protein machines, one for essentially every major process, has thereby emerged. This poses significant challenges for protein production technologies aimed at molecular level structural and functional studies of eukaryotic multiprotein complexes as intracellular quantities are typically refractory to large-scale extraction from source.

Polycistronic vectors carrying several genes encoding multiprotein complex components have been used for select cases of delivery in gene therapy (De Felipe, P. Polycistronic viral vectors. Curr. Gene Ther. 2, 355-378 (2002);Planelles, V. Hybrid lentivirus vectors. Methods Mol. Biol. 229, 273-284 (2003)). Recently, a polycistronic vector has been employed for the expression of a transcription factor complex composed of four subunits in *E. coli* (Selleck et al. A histone fold TAF octamer within the yeast TFIID transcriptional coactivator. Nat. Struct. Biol. 8, 695-700 (2001)). The DNA constructs in these studies were generated by conventional methods using endonucleases and ligase. Protein complexes in eukaryotes, however, often contain ten or more subunits with individual polypeptides ranging in size up to several hundred kDa, which severely restricts the applicability of conventional cloning strategies and largely rules out *E. coli* as a useful host system for heterologous protein expression.

Recombinant baculoviruses are particularly attractive for high-level production of large protein assemblies (O'Reilly et al. Baculovirus expression vectors. A laboratory manual. Oxford Press, New York (1994)). Genes driven by *Autographa californica* nuclear polyhedrosis virus (AcNVP) late promoters are often abundantly expressed, authentically processed, and targeted to their appropriate cellular compartment. Even architecturally complex particles such as capsid structures have been successfully assembled in insect cells using the baculovirus system (Roy et al. Baculovirus multigene expression vectors and their use for understanding the assembly process of architecturally complex virus particles. Gene 190, 119-129 (1997)). Expression of several genes in one cell can be achieved by co-infection with viruses carrying one foreign gene each. However, this approach inevitably leads to considerable variations in individual protein production levels as a result of statistical variance in virus stochiometry between infected cells. A superior alternative is infection with one baculovirus containing all heterologous genes of choice (Roy et al., see above; Bertolotti-Ciarlet et al. Immunogenicity and protective efficacy of rotavirus 2/6-virus-like particles produced by a dual baculovirus expression vector and administered intramuscularly, intranasally, or orally to mice. Vaccine 21, 3885-3900 (2003)). This is possible due to the flexible nature of the AcNVP envelope which allows for accommodation of large DNA insertions into the circular 130 kb dsDNA baculovirus genome. Traditionally, recombinant baculovirus generation is carried out in two steps. Foreign genes are first cloned into a small transfer vector propagated in *E. coli* and then inserted into the large baculovirus genome by homologous recombination in insect cells in a reaction yielding 30 - 80 % recombinant progeny if linearized parent viral DNA is used (O'Reilly et al., see above). This procedure was substantially simplified by the introduction of a baculovirus shuttle vector (bacmid bMON14272) propagated in *E. coli* (Luckow et al. Efficient generation of infectious recombinant baculoviruses by site-specific transposon-mediated insertion of foreign genes into a baculovirus genome propagated in E. coli. J. Virol. 67, 4566-4579 (1993)). The bacmid contains the Tn7 attachment site for transposition of foreign genes from a transfer vector (Bac-to-Bac^{™}, Invitrogen) (Luckow et al., see above; Bac-to-Bac^{™} Baculovirus Expression Systems Manual, Invitrogen, Life technologies Incorporated (2000)). A bicistronic transfer vector, pFastBac^{™} Dual, containing polyhedrin (polh) and p10 late viral promoters in two separate expression cassettes with sets of restriction sites for sequential subcloning of two foreign genes for co-expression was introduced.

For the production of multiprotein complexes containing large as well as many subunits, there still is a strong need for transfer vectors that allow for assembling genes in a non-sequential, modular manner to alleviate limitations imposed by the paucity of useful restriction sites in a collection of coding sequences each encompassing several thousand base pairs.

Therefore, it is an objective of the present invention to provide new tools and methods for the rapid and flexible generation of multiprotein complexes.

There is also a need for new tools and methods for recombinantly producing multiprotein complexes of ten and more subunits with individual polypeptides ranging in size up to several hundred kDa.

It is a further objective of the present invention to provide new tools and methods for generating large multiprotein complexes with many subunits and at the same time minimizing the requirements for unique restriction sites.

A further aim of the present invention is to provide for a highly flexible system for multiprotein complex production where the encoding sequences for individual subunits can be substituted in a modular manner avoiding *de novo* regeneration of the entire ensemble of encoding sequences.

There is also a need for tools and methods where facile, modular exchange of organism-, tissue- or cell type-specific promoters is possible, for recombinantly producing a particular multiprotein complex in various organisms, eukaryotic (mammalian, yeast, etc.) and prokaryotic, or under chosen conditions (e.g. early, late, very late phases of viral infection of a host cell, etc.).

The solubility and activity of biological macromolecules is often compromised if the interacting components are produced and purified in isolation, and reconstitution under *in vitro* conditions then does not produce physiologically meaningful data. Therefore, for the investigation of the interplay between macromolecular complexes among each other, there is a need for powerful and expandable systems for enabling co-expression of two or several protein assemblies ideally in one cell, each consisting of several components.

### Summary of the invention

In a first aspect, the above mentioned objective is solved by providing a novel polynucleotide for multigene applications comprising a functional arrangement according to Fig. I said arrangement comprising
(a) at least two expression cassettes T1 - MCS1 - P1 and P2 - MCS2 - T2 in a head to head, head-to-tail or tail-to-tail arrangement, each comprising a multiple cloning site MCS1 or MCS2, flanked by a promoter P1 and a terminator sequence T1 for MCS1, and flanked by a promoter P2 and a terminator sequence T2 for MCS2,
(b) at least one multiplication module M in between the promoters P1 and P2 comprising at least 2 restriction sites A and B,
(c) at least two restriction sites X and Y each flanking one of the expression cassettes, wherein:
   (i) restriction sites A and X and B and Y are compatible, but
   (ii) the ligation products of AY and BX are not enzymatically cleaved by restriction enzymes a, b, x or y, specific for restriction sites A, B, X, and Y, and
   (iii) restriction sites A and B as well as X and Y are not compatible.

A polynucleotide according to the invention may be a DNA, an RNA, or a polynucleotide comprising one or more synthetic nucleotide analogs, preferably a DNA, more preferably a double-stranded DNA.

As used herein, the term "expression cassette" is to be understood to relate to a DNA fragment which contains a promoter sequence used to recruit the DNA transcription machinery, followed by an oligonucleotide encoding for a signal sequence to recruit the RNA translation machinery (e.g. Kozak consensus in eukaryotes or Shide-Dalgarno in prokaryotes), followed by an oligonucleotide sequence which contains at least one DNA sequence cleaved by a restriction enzyme (multiple cloning site MCS) to be used for insertion of DNA fragments encoding for gene products of choice, and a terminator sequence which directs the processes related to the end of transcription and modification of the RNA transcript (e.g. polyadenylation in eukaryotes).

As used herein, the term "multiplication module" is to be understood to relate to a set of DNA fragments placed outside of expression cassettes, and/or in between expression cassettes, that allow for iterative combination of several or many expression cassettes.

Compatible restriction sites are restriction sites that, when cleaved by the cognate enzymes, result in overhanging or recessing single-stranded regions of DNA of the same nucleotide length which are cohesive, i.e. they can anneal to each other using complementary Watson-Crick base pairing, and thus can be rejoined by ligases to yield a covalently linked functional DNA molecule. Alternatively, restriction sites that when cleaved result in blunt-ended DNA fragments, are also compatible, i.e. they can be rejoined by ligases to yield covalently linked functional DNA molecules.

Non-limiting examples of restriction endonucleases that produce compatible overhangs are SpeI/AvrII; AgeI/XmaI/SgrA; BamHI/BgIII; BsrGI/BanI; EagI/NotI; EcoRI/MfeI; NdeI/AseI; NheI/XbaI; PstI/NsiI; SalI/XhoI.

Non-limiting examples of restriction endonucleases that produce compatible blunt ends, e.g. all combinations can be ligated to each other, are BstZ17I, EcoRV, Fspl, Hpal, MluNI, Pmel, Scal, SnaBI, Stul, XmnI.

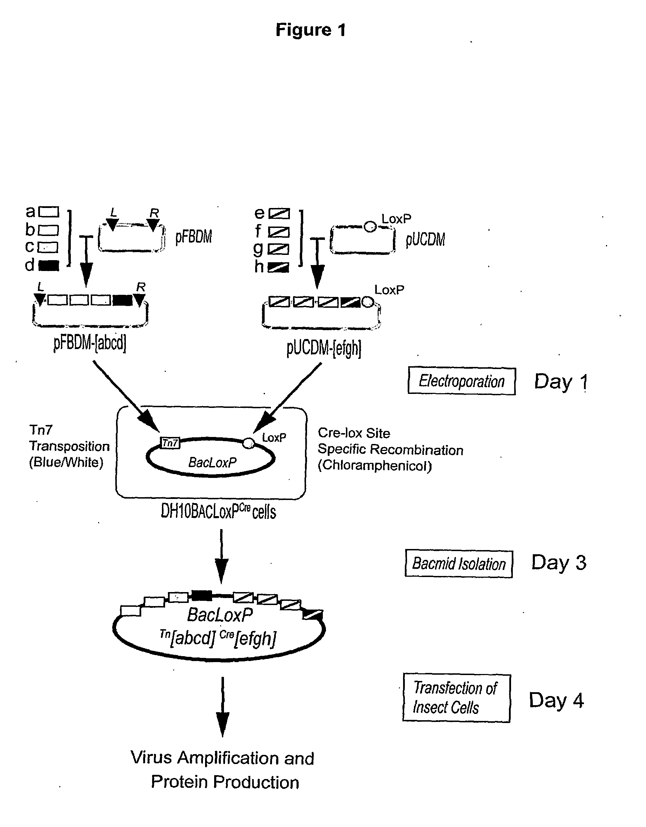

The terms "head to head", "head to tail", and "tail to tail" define the arrangement of the promoters and terminators of the individual expression cassettes towards each other. For example, T1 - T2 is a "tail to tail" arrangement, P1 - P2 is a "head to head" arrangement, and P1/P2 - T2/T1 is a "head to tail" arrangement. It is understood, that Fig. 1 shows a head to head arrangement and that, of course, different arrangements, wherein the multiplication site M is inbetween the expression cassettes and restriction sites X and Y each flank the outer bounds of the expression cassettes of the arrangement of Fig. 1 are within the scope of the invention.

Preferably, the at least two expression cassettes are arranged in a "head to head" arrangement.

In a preferred embodiment, the present invention relates to polynucleotides, wherein the restriction sites A and B in the multiplication module M are selected from the group consisting of BstZ17I, Spel, ClaI and Nrul or restriction enzymes that have identical cleavage sites as said enzymes ("isoschizomers").

In a further preferred embodiment, the present invention relates to polynucleotides, wherein the restriction sites X and Y are selected from the group consisting of Pmel and AvrII or restriction enzymes that have identical cleavage sites as said enzymes ("isoschizomers").

In another preferred embodiment, the present invention relates to polynucleotides, wherein the promoters P1 and P2 are selected from the group consisting of polh, p10 and p_{XIV} very late baculoviral promoters, vp39 baculoviral late promoter, vp39polh baculoviral late/very late hybrid promoter, P_{cap/polh}, *pcna, etl, p35, egt, da26* baculoviral early promoters; CMV, SV40, UbC, EF-1α, RSVLTR, MT, P_{DS47}, Ac5, P_{GAL} and P_{ADH}.

Furthermore, a preferred embodiment of the present invention relates to polynucleotides, wherein the terminator sequences T1 and T2 are selected from SV40, HSVtk or BGH (bovine growth hormone).

In addition to the functional arrangement of Fig. 1, it is preferred that a polynucleotide of the present invention additionally comprises at least one site for its integration into a vector or host cell. Such an integration site will allow for the convenient genomic or transient incorporation of the polynucleotide into vectors and host cells. Integration sites for genomic incorporation are more preferred.

Particularly preferred are polynucleotides, wherein the integration site is compatible for the polynucleotide's integration into a virus selected from the group consisting of adenovirus, adeno-associated virus (AAV), autonomous parvovirus, herpes simplex virus (HSV), retrovirus, rhadinovirus, Epstein-Barr virus, lentivirus, semliki forest virus and baculovirus.

More preferred are polynucleotides according to the invention, wherein the integration site is compatible for the polynucleotide's integration into a baculovirus.

The baculovirus expression system has been used extensively for the expression of recombinant proteins in insect cells. Recently, recombinant baculovirus vectors engineered to contain mammalian cell-active promoter elements, have been used successfully for transient and stable gene delivery in a broad spectrum of primary and established mammalian cell lines. The application of modified baculoviruses for in *vivo* gene delivery has also been demonstrated. In contrast to other commonly used viral vectors, baculoviruses have the unique property of replicating in insect cells while being incapable of initiating a replication cycle and producing infectious virus in mammalian cells. The viruses can be readily manipulated, accommodate large insertions of foreign DNA, initiate little to no microscopically observable cytopathic effect in mammalian cells and have a good biosafety profile (Kost et al. Recombinant baculoviruses as mammalian cell gene-delivery vectors. Trends in Biotechnology, 20(4), April 2002). These attributes make baculoviruses particularly useful for practicing the present invention.

In a more preferred embodiment, said integration site(s) optionally comprised in the polynucleotide of the present invention is(are) selected from the group consisting of the transposon elements of Tn7, λ-integrase specific attachment sites and SSRs (site specific recombinases), preferably the cre-lox specific recombination (LoxP) site or the FLP recombinase specific recombination (FRT) site.

Preferably, the integration site(s) is(are) compatible for the polynucleotide's integration into a eukaryotic host cell selected from the group consisting of mammalian, preferably human cells; porcine, preferably CPK, FS-13, PK-15 cells; bovine, preferably MDB, BT cells; ovine, preferably FLL-YFT cells; *C.elegans;* yeast, preferably *S.cerevisiae, S.pombe, C.albicans, P. Pastoris cells*; and insect cells.

For human host cells, the integration site is preferably compatible for the polynucleotide's integration into a host cell selected from the group consisting of HeLa, Huh7, HEK293, HepG2, KATO-III, IMR32, MT-2, pancreatic β-cells, keratinocytes, bone-marrow fibroblasts, CHP212, primary neural cells, W12, SK-N-MC, Saos-2, WI38, primary hepatocytes, FLC4, 143TK-, DLD-1, embryonic lung fibroblasts derived from human foetus, primary foreskin fibroblasts, Saos-2 osteosarcoma, MRC5, and MG63 cells.

For insect host cells, the integration site is preferably compatible for the polynucleotide's integration into a host cell selected from the group consisting of *S. frugiperda* cells, more preferably Sf9, Sf21, Express Sf+, High Five H5 cells, and *D. melanogaster* cells, most preferably S2 Schneider cells.

In a preferred embodiment, the polynucleotide according to the invention additionally comprises one or more resistance markers for selecting host cells with desired properties based on their resistance to toxic substances. More preferably, said resistance markers are selected from ampicillin, chloramphenicol, gentamycin, spectinomycin, and kanamycin resistance markers.

When a prokaryotic host cell is desired for incorporating a nucleotide of the present invention, it is preferred that the polynucleotide according to the invention additionally comprises a conditional R6Kγ origin of replication for making propagation dependent on the *pir* gene in a prokaryotic host.

In a very preferred embodiment, the polynucleotide according to the invention comprises
(a) promoters P1 and P2 selected from polh and p10,
(b) terminator sequences selected from SV40 and HSVtk,
(c) restriction sites A and B in the multiplication module M selected from the group consisting of BstZ17I, Spel, ClaI and Nrul,
(d) restriction sites X and Y selected from the group consisting of Pmel and AvrII,
(d) sites for virus integration selected from the cre-lox and Tn7 recombination system.

In a further aspect, the present invention is directed to a vector comprising a polynucleotide sequence according to the present invention.

In a preferred embodiment said vector is selected from the group consisting of plasmids, expression vectors, and transfer vectors.

These vectors are useful for the incorporation of heterologous DNA elements, preferably genes. Said vectors may be expression vectors or transfer vectors, preferably useful for eukaryotic gene transfer, transient or viral vector mediated gene transfer.

More preferred said vector is a plasmid or a virus.

In a specifically preferred embodiment, said virus is selected from the group consisting of adenovirus, adeno-associated virus (AAV), autonomous parvovirus, herpes simplex virus (HSV), retrovirus, rhadinovirus, Epstein-Barr virus, lentivirus, semliki forest virus and baculovirus.

In a most preferred embodiment, the vector of the present invention is a baculovirus expression vector.

When engineering baculovirus expression vectors, it was surprisingly found that the functional disruption of two baculoviral genes, v-cath and chiA, leads to improved maintenance of cellular compartiments during infection and protein expression. The quality of the proteins produced by such a novel baculovirus system is significantly improved through reduction of viral-dependent proteolytic activity and cell lysis.

Therefore, in a different aspect, the present invention is also directed to baculoviruses in general, wherein the two baculoviral genes v-cath and chiA are functionally disrupted.

In a preferred embodiment, the present invention is directed to a baculovirus vector according to the invention, wherein the two baculoviral genes v-cath and chiA are functionally disrupted.

Preferably, vectors according to the present invention additionally comprise a site for SSRs (site specific recombinases), preferably LoxP for cre-lox site specific recombination.

More preferably, the cre-lox site is located in one or both of the two baculoviral genes v-cath and chiA and disrupts their function.

In another preferred embodiment, the present invention relates to vectors comprising a transposon element, preferably the Tn7 attachment site.

Preferably, said attachment site is located within a marker gene. That way successful integration by transposition can be assessed through the marker gene.

More preferably, the marker gene is selected from the group consisting of luciferase, β-GAL, CAT, fluorescent protein encoding genes, preferably GFP, BFP, YFP, CFP and variants thereof, and the lacZα gene. Variants of marker genes are those that deviate in sequence by less than 30, preferably less than 20, more preferably less than 10 % but retain substantially the same functional marker properties as the original marker gene.

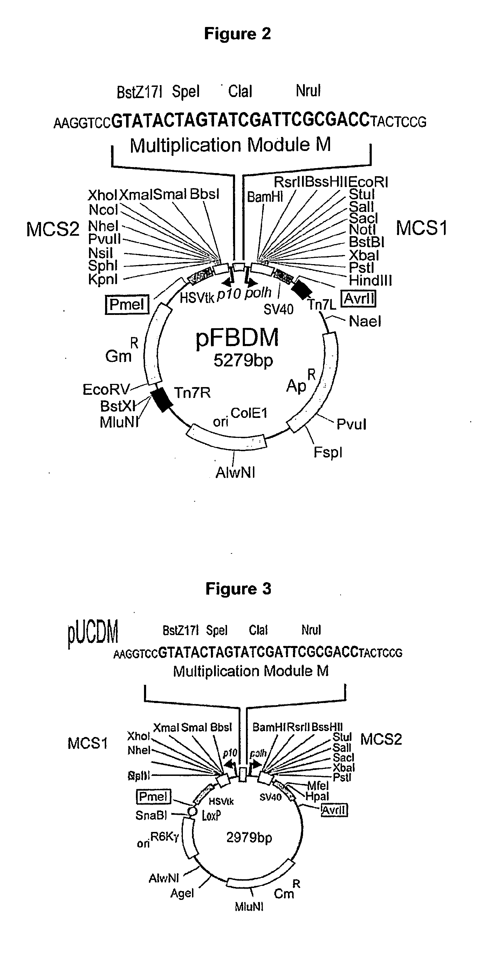

In a most preferred embodiment, the vector of the present invention has the sequence according to SEQ ID NO: 1 (pFBDM). See also Fig. 2.

In a further most preferred embodiment, the vector of the present invention has the sequence according to SEQ ID NO: 2 (pUCDM). See also Fig. 3.

In the following, the vectors of the present invention will be exemplified with reference to the most preferred baculoviral vectors pFBDM and pUCDM. However, the following description of these specific vectors of the present invention is not to be construed as limiting the scope of the invention.

The above described preferred new baculovirus transfer vectors were constructed specifically for multigene applications. They comprise modified recipient baculovirus DNA engineered for improved protein production, and allow for a simple and rapid method to integrate genes via two access sites (attTn7 and LoxP) into this baculoviral DNA in *E. coli* cells tailored for this purpose.

The vectors of the present invention allow for the elucidation of protein interaction networks (interactome). Since many of the identified multiprotein complexes are not present in sufficient quantities in their native cells for detailed molecular biological analysis, their study is dependent on recombinant technologies for large-scale heterologous protein production. Currently, recombinant expression methods require a disproportionate investment in both labor and materials prior to multiprotein expression, and subsequent to expression do not provide flexibility for rapidly altering the multiprotein components for revised expression studies.

The vectors of the present invention, in particular baculovirus expression systems according to the invention, facilitate and accelerate multiprotein expression immensely.

The transfer vectors of the invention, in particular pFBDM and pUCDM, that contain a multiplication module, greatly facilitate modular combination of heterologous genes with a minimum requirement for unique restriction sites. Viral promoters (e.g. p10 and polh very late promoters) in these vectors can be exchanged to other promoter sequences (early, late) if required. Likewise, terminator sequences (currently SV40, HSVtk) can be substituted.

The use of baculoviral vectors with functionally disrupted v-cath and chiA genes allows for improved maintenance of cellular compartments during infection and protein production. The quality of proteins produced by such a vector system is significantly improved through a reduction of viral dependent proteolytic activity and reduced cell lysis.

Furthermore, the vectors of the present invention allow for a completely new protocol for rapid combinatorial generation of recombinant DNA, preferably baculoviral DNA, by accessing the vector genome via two specific sites.

In addition to a Tn7 transposon site, a LoxP sequence was introduced at a separate site on the baculovirus genome which can accept multigene expression cassettes from the pUCDM plasmid by site-specific recombination. A protocol was developed for carrying out Tn7 transposition (from pFBDM derivatives carrying multigene cassettes) and LoxP site-specific recombination (from pUCDM derivatives carrying multigene cassettes) efficiently in a single step in *E.coli*. These protocols can be used not only to integrate multigene cassettes with coding sequences for multiprotein complex subunits into vectors (e.g. baculovirus vectors), but also to integrate specific enzymes (kinases, acetylases etc.) for modifying the proteins under investigation. See Fig. 1 for further illustration.

The transfer vector pFBDM contains two expression cassettes in a head-to-head arrangement with multiple cloning sites MCS1 and MCS2 flanked by polh or p10 promoters and SV40 or HSVtk polyA signal sequences, respectively. Multiplication module M is located in between the promoter sequences. The sequences used for Tn7 transposition (Tn7L and Tn7R) encompass the expression cassettes and a gentamycin resistance marker. (See Fig. 2 for more detail.)

The transfer vector pUCDM has an identical expression cassette including a multiplication module the same as for pFBDM. This expression cassette is flanked by a LoxP inverted repeat. Vector pUCDM contains a chloramphenicol resistance marker and a conditional R6Kγ origin of replication which makes its propagation dependent on the expression of the *pir* gene in the prokaryotic host. See Fig. 3 for more detail.

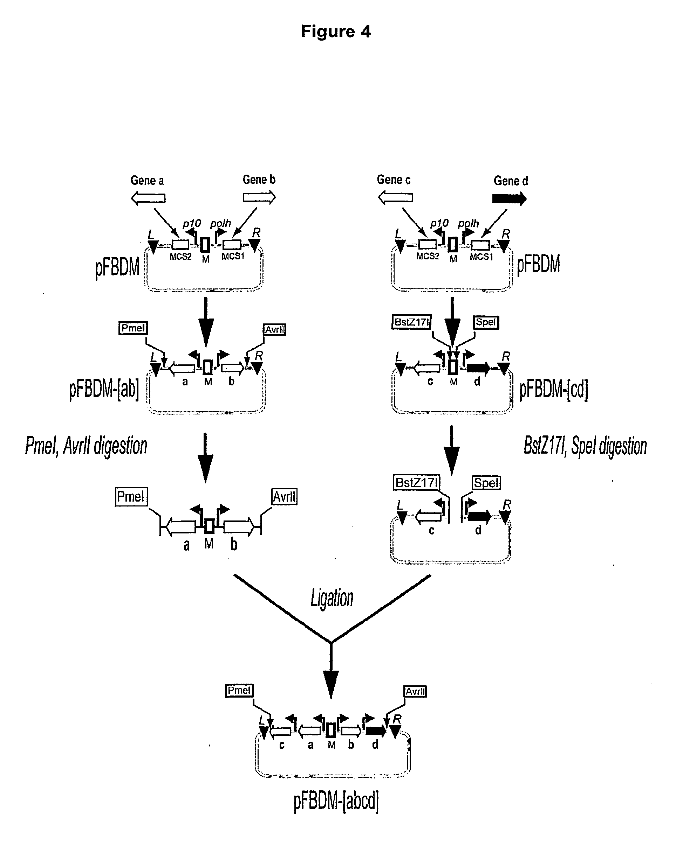

The vectors pFBDM and pUCDM are particularly suited for generating multigene expression cassettes due to the multiplication module inserted in between the two promoters. The logic of multiplication is illustrated in Fig. 4. The only prerequisite for assembling multigene expression cassettes is that the restriction enzymes used for multiplication (e.g. Pmel, AvrII, Spel, and either BstZ17I or Nrul) are unique, which can be easily accomplished for instance by site directed mutagenesis prior to multigene cassette assembly. Genes are cloned into MCS1 and MCS2 of pFBDM. The entire expression cassette is then excised by Pmel and AvrII digestion. The resulting fragment is placed into the multiplication module of a pFBDM derivative containing further sets of genes via either SpeI/BstZ17I or SpeI/NruI sites. Spel produces a cohesive end compatible with AvrII, while BstZ17I, Nrul and Pmel are blunt-cutters). The restriction sites involved are eliminated in the process and multiplication can be repeated iteratively using the module present in the inserted cassette. The same logic applies also for generation of pUCDM derivatives with multigene expression cassettes. Also, promoter and terminator sequences can be easily modified if desired using appropriate restriction sites in these vectors. See Fig. 4 for more detail.

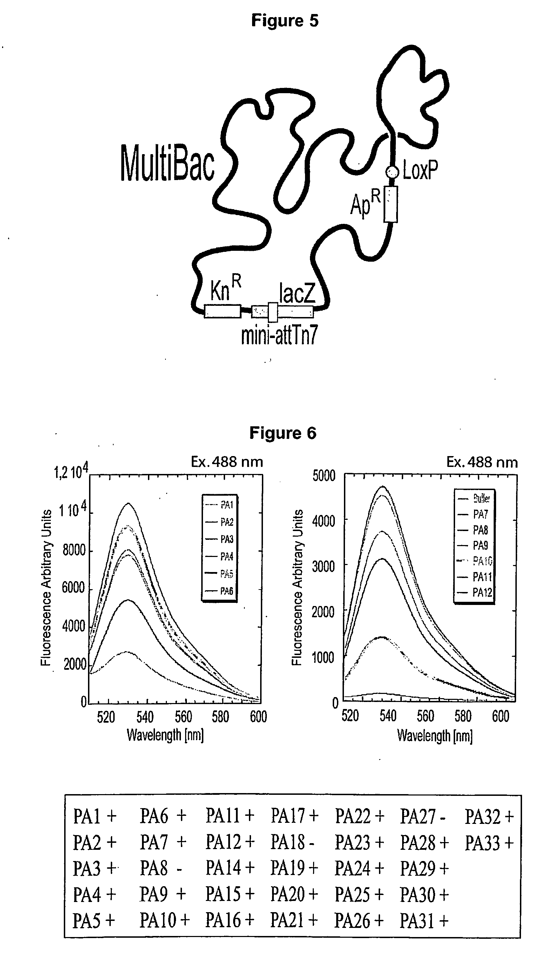

In addition, the baculovirus genome was modified to obtain improved protein production properties. Two baculoviral genes, *v-cath* and *chiA,* were disrupted which leads to improved maintenance of cellular compartments during infection and protein production. The *v-cath* gene encodes for a viral protease, V-CATH, which is activated upon cell death by a process dependent on a juxtaposed gene on the viral DNA, *chiA,* which encodes for a chitinase. Both genes were disrupted to eliminate V-CATH activity and to gain the option of utilizing chitin-affinity chromatography for purification without interference from the *chiA* gene product. The quality of proteins produced by the MultiBac baculovirus is significantly improved through a reduction of viral-dependent proteolytic activity and reduced cell lysis. In place of the disrupted viral DNA sequence, a LoxP sequence for *cre-lox* site-specific recombination was placed. See Fig. 5 for more detail.

As used herein, the term "MultiBac" stands synonymously for the exemplary and preferred embodiment of the said baculovirus viral vector, i.e. a modified baculovirus genome lacking the genes for *v-cath* and *chiA.* In addition, "MultiBac" in the context of "MultiBac system" encompasses said modified baculovirus viral vector as well as the *E.coli* cell types derived from the DH10α strain which contain said baculovirus vector (DH10MultiBac^{ET}, DH10MultiBac^{Cre} etc.) along with the factors required for carrying out the recombination reactions described herein (Tn7 transposition, Site specific recombination) and additionally the exemplary and preferred vectors, pFBDM and pUCDM, according to the present invention.

All vectors of the present invention, in particular the preferred transfer vectors pFBDM and pUCDM, are useful for introducing multiprotein complexes into host cells.

In another aspect, the present invention relates to a host cell comprising a polynucleotide and/or a vector according to the invention.

Preferred host cells for practicing the invention are those selected from the group consisting of mammalian cells, preferably human cells, rodent cells, porcine cells, bovine cells, ovine cells; *C. elegans* cells; yeast cells; insect cells; and *E. coli* cells.

Preferred yeast cells for practicing the invention are *S. cervisiae, S.pombe, C. albicans* and *P. pastoris* Preferred *E. coli* cells for practicing the invention are Top10, DH5α, DH10α, HB101,

TG1, BW23473, BW23474 cells.

Preferred insect cells for practicing the invention are *S. frugiperda* cells, preferably Sf9, Sf21, Express Sf+ or High Five H5 cells, and *D. melanogaster,* preferably S2 Schneider cells.

Preferred human cells for practicing the invention are selected from the group consisting of HeLa, Huh7, HEK293, HepG2, KATO-III, IMR32, MT-2, pancreatic β-cells, keratinocytes, bone-marrow fibroblasts, CHP212, primary neural cells, W12, SK-N-MC, Saos-2, WI38, primary hepatocytes, FLC4, 143TK-, DLD-1, embryonic lung fibroblasts derived from human foetus, primary foreskin fibroblasts, Saos-2 osteosarcoma, MRC5, and MG63 cells.

Of course, cells comprising a polynucleotide and/or a vector according to the present invention are by no means limited to isolated cells or cell tissues.

In another aspect, the present invention also relates to a recombinant non-human animal comprising a polynucleotide sequence and/or a vector according to the invention.

Such recombinant non-human animals are useful for elucidating the role of multienzyme complexes or screening compounds for biological activities *in vivo.*

Preferably, non-human animals according to the invention are selected from mammals, preferably rodent, porcine and bovine; *C.elegans*; and insects, preferably *S. frugiperda* and *D. melanogaster.*

The tools of the present invention, i.e. the polynucleotides, vectors, host cells and recombinant animals, allow for the convenient, rapid and simple modular combination of heterologous genes with a minimum requirement for unique restriction sites.

In another aspect, the present invention is directed to a method for generating multigene expression cassettes, comprising the steps of
(a) cloning one or more genes into MCS1 and/or MCS2 of a first vector according to the invention,
(b) cloning one or more genes into MCS1 and/or MCS2 of a second vector according to the invention,
(c) excising the entire functional arrangement at the restriction sites X and Y in the first transfer vector,
(d) cutting the second transfer vector at the multiplication module M,
(e) ligating the excised functional arrangement of step (c) into the cut multiplication site of the second vector of step (d), thereby producing a third transfer vector having the multiplication module of the first transfer vector and expression cassettes of both the first and the second vector, and
(f) optionally repeating steps (a) through (e) for assembling a transfer vector with multiple expression cassettes.

Preferably, the restriction sites X and Y in step (c) are Pmel and AvrII or isoschizomers of these enzymes and the restriction sites at the multiplication module of the second vector in step (d) are selected from BstZ17I, Spel, Clal and Nrul or isoschizomers of these enzymes.

Most preferably, baculovirus transfer vector pFBDM can be employed for step (a) and/or (b).

Also, most preferably, baculovirus transfer vector pUCDM can be employed for step (a) and/or (b).

For a better understanding of the method of the invention in general, an exemplary method using preferred vector pFBDM is illustrated in Fig. 4.

The method of the invention allows for the conventional introduction of genes into one or more expression cassettes of vectors of the invention and subsequently facilitates the modular assembly of these expression cassettes into a single vector while at the same time minimizing the number of required restriction sites to do so.

A further aspect of the present invention is directed to a method for producing multiprotein complexes *in vitro* comprising the steps of
(a) generating multigene expression cassettes according to the invention as described above,
(b) transferring the multigene expression cassettes into a host cell capable of expressing said genes simultaneously.

Preferably, the transfer in step (b) is effected by means of a vector, preferably a virus.

Preferably, the host cell is selected from mammalian, yeast, E. coli, and insect cells.

When the host cell is an insect cell, it is preferably *S. frugiperda* cells, more preferably Sf9, Sf21, Express Sf+ or High Five H5 cells, or *D. melanogaster* cells, more preferably S2 Schneider cells.

When the host cell is a mammalian cell, it is preferably a human cell selected from the group consisting of HeLa, Huh7, HEK293, HepG2, KATO-III, IMR32, MT-2, pancreatic β-cells, keratinocytes, bone-marrow fibroblasts, CHP212, primary neural cells, W12, SK-N-MC, Saos-2, WI38, primary hepatocytes, FLC4, 143TK-, DLD-1, embryonic lung fibroblasts derived from human foetus; primary foreskin fibroblasts, Saos-2 osteosarcoma, MRC5, and MG63 cells.

When the host cell is a yeast cell, it is preferably *S. cerevisiae, S.pombe, C.albicans* or *P. pastoris.*

The present disclosure is not limited to *in vitro* methods.

A different aspect of the present disclosure relates to a method for producing multiprotein complexes *in vivo* comprising the steps of
(a) generating multigene expression cassettes according to claims 39 to 42,
(b) transferring the multigene expression cassettes into an animal wherein said genes are expressed simultaneously.

Preferably, the transfer in step (b) is effected by means of a vector, preferably a virus vector, more preferably a baculovirus vector.

As mentioned above, baculoviruses are particularly useful as mammalian cell gene-delivery vehicles.

For producing multiprotein complexes *in vivo,* said animal is preferably selected from mammals, *C.elegans,* or insects.

Preferred insects for practicing said method are *S. frugiperda* and *D. melanogaster.*

Preferred mammals for practicing the invention are human, rodent, porcine, or bovine.

The recent discovery of an ever increasing number of multiprotein complexes in eukaryotic cells (the 'interactome'), calls for improved heterologous expression techniques in particular for protein complexes with intracellular quantities refractory to extraction from source (e.g. close to all *human* multiprotein complexes). Eukaryotic proteins are often large (>100 kDa) multidomain entities and exist in the cell as complexes of on average 5 to 8 proteins. In general, protein complexes containing large proteins can not be produced in a functionally active state with generic (e.g. prokaryotic) expression technologies. Further, components of multiprotein assemblies often display folding problems and compromised biological activity when produced in isolation. Current eukaryotic expression technologies (baculovirus system, yeast and mammalian expression systems etc.) are not designed for rapid and flexible expression of several large genes encoding for multiprotein complexes.

The vectors of the present invention, however, are tailored to meet these requirements. Said vectors are capable of carrying multiple gene expression cassettes for insertion into e.g. a modified baculovirus with improved protein production properties via independent entry sites (*Cre-loxP* and *Tn7 and*/*or others*).
In combination with the high-level production rates obtained for example in insect cells, vectors of the present invention can thus be used in a flexible manner for the generation of multiprotein complexes for structural and functional studies providing significant information for use in research and drug development, for instance as physiologically meaningful targets for discovery of ligands that influence their activity (inhibitors, antibodies etc.).

The disclosure is also particularly useful for preparing a vaccine directed against multi subunit protein complexes in mammals. Complex multiprotein units will often display different epitopes in comparison to individual proteins and will therefore resemble the relevant epitopes of many naturally occurring protein complexes more closely, thus providing better antigen targets for antibody production. In addition, multiple proteins for multivalent vaccines and optionally even protein adjuvants can be introduced in only one vector and be expressed simultaneously to assure maximum efficacy.

Recently, virion-like particles (VLPs) consisting of four proteins from the severe acute respiratory syndrome (SARS) coronavirus were made using a recombinant baculovirus expression vector (Mortola et al. Efficient assembly and release of SARS coronavirsus-like particles by a heterologous expression system FEBS Lertters 576, 174-178 (2004)), offering a potential candidate vaccine for this disease. The use of vectors according to the present invention, due to their modular ability to carry multiple gene expression units, will greatly facilitate the generation of such vaccines, consisting of even more numerous components than SARS-VLPs.

Therefore, the present disclosure also relates to a method for producing a vaccine comprising the steps of
(a) administering at least one polynucleotide and/or vectors according to the invention to a mammal, whereby at least one protein coded for by at least one expression cassette of said polynucleotide is expressed within the animal,
(b) optionally administering an adjuvans to said mammal,
(c) optionally isolating the antibodies and or spleen cells producing antibodies specific for said at least one protein.

The present invention provides for a convenient and simple protocol for recombinantly producing multiprotein complexes found in nature. These multiprotein complexes can be assayed for protein complex interactions or modifications of the proteins. They can also be assayed for interaction with compounds suspected of biological activity or even medical value.

In another aspect the present disclosure is directed to a method for assaying protein complex interactions or modifications of proteins.

In a preferred embodiment, the present disclosure relates to a method for the *in vitro* screening for protein complex interactions or modifications of proteins, comprising the steps of
(a) providing a host cell comprising at least one polynucleotide and/or a vector according to the invention, comprising at least one expression cassette with genes for at least two proteins of interest and capable of expressing said proteins simultaneously,
(b) detecting interactions or modifications in one or more of the expressed proteins.

In another preferred embodiment, the present disclosure encompasses a method for the *in vitro* screening of candidate compounds capable of
(i) protein complex interactions or (ii) modification(s) of protein(s) (of a multiprotein complex) or (iii) capable of inhibiting protein complex interactions or (iv) inhibiting modification(s) of protein(s), comprising the steps of
   (a) providing a host cell comprising at least one polynucleotide according to the invention and/or a vector according to the invention, comprising at least one expression cassette with genes for at least two proteins of interest and capable of expressing said proteins simultaneously,
   (b) administering the candidate compound to the host cell of step (a),
   (c) detecting protein complex interactions or modifications in one or more of the expressed proteins or detecting the inhibition of protein complex interactions or the inhibition of modifications of proteins.

The present disclosure also encompasses *in vivo* screening assays.

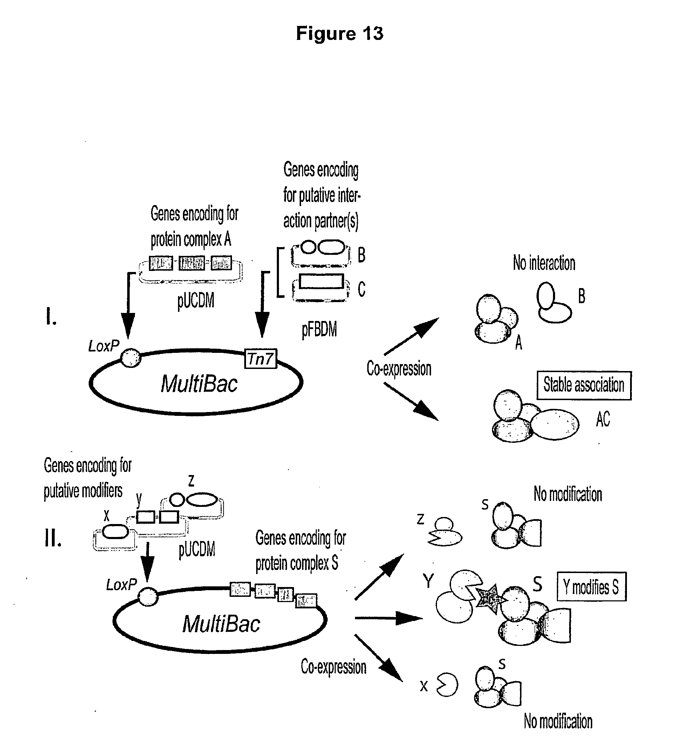

The polynucleotides and/or vectors of the present invention can also be used for the *in vivo* screening of protein - protein or multiprotein complex - multiprotein complex interactions (See Fig. 13 I. for a general illustration of such a screening method), also from randomized libraries or cDNA pools as well as physiological modifications (phosphorylation, glycosylation etc.) of particular protein complexes or multiprotein assemblies (see Fig. 13 II. for a general illustration of such a screening method).

In a further preferred embodiment, the present disclosure teaches a method for *in vivo* screening of candidate compounds capable of (i) protein complex interactions or modification(s) of protein(s) (of a multiprotein complex) or (iii) capable of inhibiting protein complex interactions or inhibiting modification(s) of protein(s), comprising the steps of
(a) providing an animal comprising at least one polynucleotide according to the invention and/or a vector according to the invention, comprising at least one expression cassette with genes for at least two proteins of interest and capable of expressing said proteins simultaneously,
(b) administering the candidate compound to the animal of step (a),
(c) detecting protein complex interactions or modifications in one or more of the expressed proteins or detecting the inhibition of protein complex interactions or the inhibition of modifications of proteins.

Bioactive multiprotein complexes but also medically advantageous combinations of proteins such as e.g. antibody mixtures, optionally with interleukins and/or adjuvants can be administered to mammals simultaneously by means of the polynucleotides of the present invention and/or gene-delivery vectors of the present invention.

Therefore, in a further aspect, the present disclosure also relates to the use of a polynucleotide and/or a vector according to the invention for the preparation of a medicament comprising a multigene transfer vehicle for gene therapy.

Tremendous efforts are being made to develop gene delivery systems for therapeutic applications. Gene therapy has been the focus of intense enthusiasm but also critique in the past. To date, major progress has been made in evaluating gene therapy in clinical trials on the way to achieving safe and applicable clinical *in vivo* and ex *vivo* strategies for human diseases (Worgall S. A realistic chance for gene therapy in the future. Pediatr. Nephrol. (2004)). Overall, gene therapy now stands as a very promising avenue for the correction of genetic as well as acquired disorders entailing permanent or transient expression of a therapeutic gene product (Worgall S. see above). Recombinant virus based vectors, in particular those that are not replication-competent in mammalian hosts (e.g. baculoviral vectors), have emerged recently as a powerful tool for mammalian cell gene delivery and successfully applied to a whole range of mammalian cell lines, including human, primates, rodent, bovine, procine and ovine (reviewed in Kost and Condreay. Recombinant baculoviruses as mammalian cell gene-delivery vectors. Trends Biotechnol. 20, 173-180 (2002)). To obtain complex gene transfer/therapy effects, both *ex vivo* and *in vivo*, an increasing demand has arisen for polycistronic viral vectors to accomplish more powerful results by combined gene therapy than from single gene therapy (de Felipe, P. Polycistronic viral vectors. Curr. Gene Ther. 2, 355-378 (2002); Planelles, V. Hybrid lentivirus vectors. Methods Mol. Biol. 229, 273-284 (2003)). The requirement for the incorporation of accessory genes into a carrier virus that is to be administered *in vivo,* for instance to block inactivation by the complement system, has also been demonstrated by using a pseudotyped baculovirus with baculoviral gp64 envelope proteins that carried a human decay-accelerating factor protein domain fusion (Hueser et al., Incorporation of decay-accelerating factor into the baculovirus envelope generates complement-resistant gene transfer vectors. Nat. Biotechnol. 19, 451-455 (2001)), exemplifying the necessity to provide recombinant modifications on the virus production level in addition to the multiple genes to be transferred for therapeutic purposes.

Preferably, the vector used for preparing a medicament is a recombinant baculovirus.

More preferably, the vector used for preparing a medicament disclosed herein is a baculovirus comprising at least one multigene expression cassette coding for
(i) one or more gene products, preferably proteins, for therapy, and
(ii) one or more baculovirus proteins,
and wherein the baculovirus is capable of expressing the genes for the therapeutic gene products, preferably proteins, under the control of mammalian promoter(s) active in the animal being treated, and the genes for the baculovirus proteins under the control of baculovirus promoters.

In a preferred embodiment, the protein(s) of step (ii) are humanized baculoviral proteins expressed from a pseudotyped baculovirus, preferably a humanized baculovirus envelope protein gp64, e.g. gp64 fused with a human protein such as for example decay accelerating factor.

In a more general aspect the present invention is directed to the use of a polynucleotide and/or a vector and/or a host cell and/or an animal according to the invention for the recombinant production of multiprotein complexes.

Using genes encoding for fluorescent proteins EYFP and ECFP a close to linear increase of fluorescent protein production with the number of genes encoding for this protein that were integrated in vectors of the present invention was surprisingly seen. More precisely, the production yield (per 1 L insect cell culture) of fluorescent protein was 6-8 mg/L if one copy of the gene was present, about 11 mg if two copies were present, and about 18 mg if three copies of the gene were present in a vector of the present invention used for expression. This suggests a previously unnoticed, simple way to dramatically enhance the quantity of a recombinant protein expressed using an expression system according to the invention, namely by simply providing two, three or more copies of the same gene instead of only one. This is of special interest for industrial scale protein production for instance for pharmaceutical purposes, in particular for membrane proteins which are of paramount interest for drug development (e.g. GPCRs), the production of which is currently largely hampered by the low production yields.

Also, the recombinant multiprotein complexes produced according to the present invention are particularly useful for biophysical, structural, crystallographical, electron-microscopical, and NMR based analysis of multiprotein complexes for research and also for drug development.

Preferably, the disclosure relates to the use of a polynucleotide and/or a vector and/or a host cell and/or an animal according to the invention for producing a vaccine.

Also preferred is the use of a polynucleotide and/or a vector and/or a host cell and/or an animal disclosed herein for screening at least one protein *in vitro* or *in vivo*, optionally together with a candidate compound, for protein association, protein modification, or a biological effect in a host cell or animal or for the inhibition of protein association, protein modification, or a biological effect in a host cell or animal.

In another aspect, the present disclosure relates to a kit of parts comprising at least a polynucleotide and/or a vector and/or a host cell according to the invention, optionally also comprising an instruction manual, antibiotics, buffers, and/or marker compounds.

### Description of the figures

### Figure 1: The MultiBac system in a schematic view.

Genes of interest are assembled into multigene expression cassettes using the multiplication module present on transfer vectors pFBDM and pUCDM. The resulting vectors are introduced into MultiBac baculoviral DNA in DH10MultiBac^{Cre} *E. coli cells* which contain the factors for Tn7 transposition (for pFBDM derivatives) and *cre-lox* site-specific recombination (for pUCDM derivatives). Colonies containing bacmid carrying integrated multigene cassettes are identified by blue/white screening (Tn7 transposition disrupts a lacZα gene) and chloramphenicol resistance (conferred by Cre catalyzed integration of pUCDM derivative). Transposition and site-specific recombination are carried out either sequentially or, alternatively, concomitantly in a one-step reaction. Bacmid DNA is prepared from selected clones and used to transfect insect cells for protein production.

### Figure 2: Transfer vector pFBDM.

The circle map of pFBDM shows promoters (polh, p10), terminators (SV40, HSVtk), multiple cloning sites (MCS1, MCS2), transposon elements (Tn7L, Tn7R) and resistance markers (ampicillin and gentamycin). Genes of interest are cloned into MCS1 or MCS2 using unique restriction sites. The multiplication module (M) is located in between the p10 and polh promoters.

### Figure 3: Transfer vector pUCDM.

The circle map of pUCDM shows promoters (polh, p10), terminators (SV40, HSVtk), multiple cloning sites (MCS1, MCS2), the inverted repeat for *cre-lox* site-specific recombination (LoxP) and a resistance marker (chloramphenicol). Genes of interest are cloned into MCS1 or MCS2 using unique restriction sites. The multiplication module (M) is located in between the p10 and polh promoters.

### Figure 4: Assembling multigene expression cassettes.

The logic of multiplication is shown for pFBDM. The expression cassette containing two genes of choice (denoted a,b) is excised by digestion with AvrII and Pmel (boxed) and placed into a multiplication module of a construct containing further genes (c,d) via BstZ17I/SpeI (or, alternatively NruI/SpeI) sites present in the multiplication module (M). Spel produces a cohesive end compatible with AvrII, while BstZ17I, Nrul and Pmel are blunt-cutters. Multigene derivatives of pUCDM can be generated following the same logic.

### Figure 5: MultiBac baculoviral DNA.

The modified viral genome is shown in a schematic representation. The Tn7 attachment site is located within a LacZα gene; insertion of Tn7 elements from pFBDM derivatives therefore produces a white phenotype when plated on agar containing BluoGal and IPTG. In place of the disrupted *v-cath* and *chiA* viral genes, a LoxP sequence was inserted to accept pUCDM derivatives by Cre catalysis, producing a chloramphenicol resistant phenotype.

### Figure 6:. Efficiency of cre-lox site-specific recombination in MultiBac.

The gene encoding for yellow fluorescent protein EYFP was cloned into pUCDM and integrated into MultiBac by Cre catalysis. Virus was clonally separated by plaque assay. 32 plaques were tested for EYFP expression by fluorescence spectroscopy. Excitation was at 488 nm. Spectra recorded from cell lysate from viruses 1 to 12 are shown (above). In this experiment, 91% (29 of 32) of the specimens showed strong fluorescence emission indicating expression of the EYFP protein (Table below).

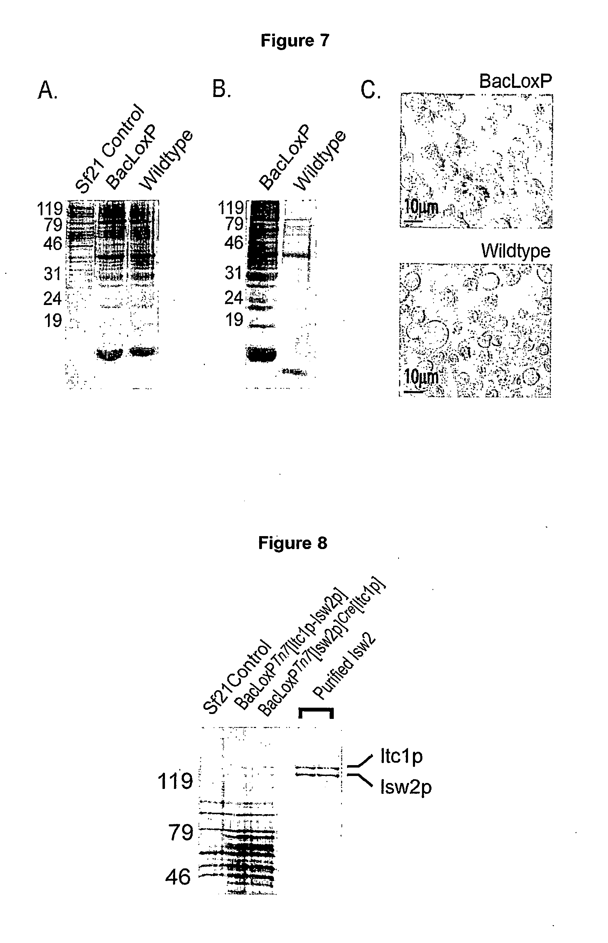

### Figure 7: Effect of v-cath and chiA deletion in MultiBac.

Comparison of the lysate from cells infected with MultiBac virus and a commercially available baculovirus ("Wildtype"). Viral-dependent protein production in both samples is virtually identical at 48 hours post infection (Fig. A). Incubation of cell lysate for 96 hours at RT after harvest shows a virtually unaltered protein content in the sample from cells infected with MultiBac. In contrast, proteolysis is evident in the sample from cells infected with wildtype virus (Fig. B). Effect of protease deletion on cell morphology at 72 hours post infection is shown in micrographs of cells infected with MultiBac lacking *v-cath* and *chiA* (MultiBac) or with commercial virus containing the protease and chitinase genes (Wildtype). Cells in the upper micrograph are uniformly round and appear intact. In contrast, cell lysis is prevalent in the lower micrograph (Fig. C).

### Figure 8: Expression of the 275 kDa chromatin remodeling complex Isw2

### using MultiBac.

Isw2 complex was expressed from the attTn7 site or, alternatively, from the attTn7 and LoxP sites of MultiBac. Cell lysate as well as protein complex purified from both composite bacmids exhibits virtually identical protein production levels. Sample from uninfected Sf21 cells is included as a control.

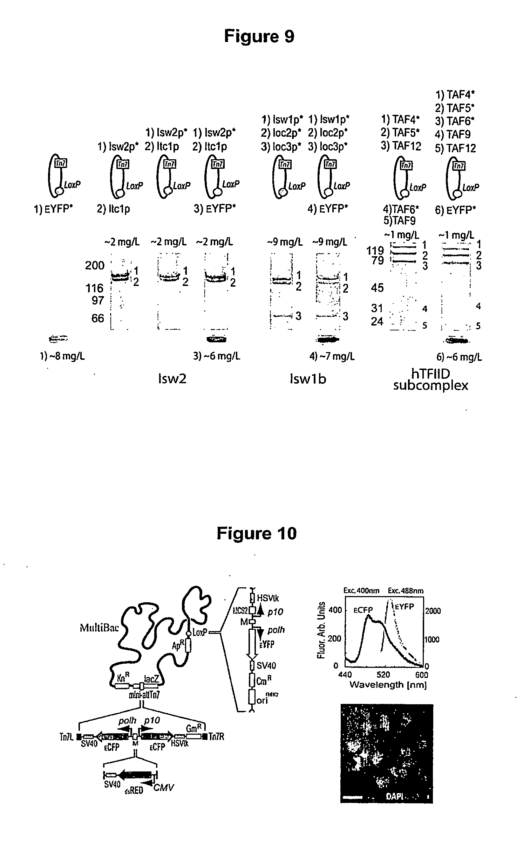

### Figure 9: Expression of yeast Isw2, Isw1b and a human TFIID subcomplex using MultiBac.

Integration sites are depicted schematically on top of Coomassie stained gel sections showing the components of the respective multiprotein complexes. Yields are indicated. Expression of EYFP is shown below by Western Blot sections of the respective cell lysates, with yields calculated from absorbance spectra.

### Figure 10: Use of MultiBac for gene transfer in mammalian cells ("BacMam").

A hybrid virus (left) results in expression of EYFP and ECFP in insect cells (right, top) and dsRED expression in COS cells infected with virus amplified in Sf21 cells (right, bottom).

### Figure 11: Circular baculovirus transfer vector pFBDM

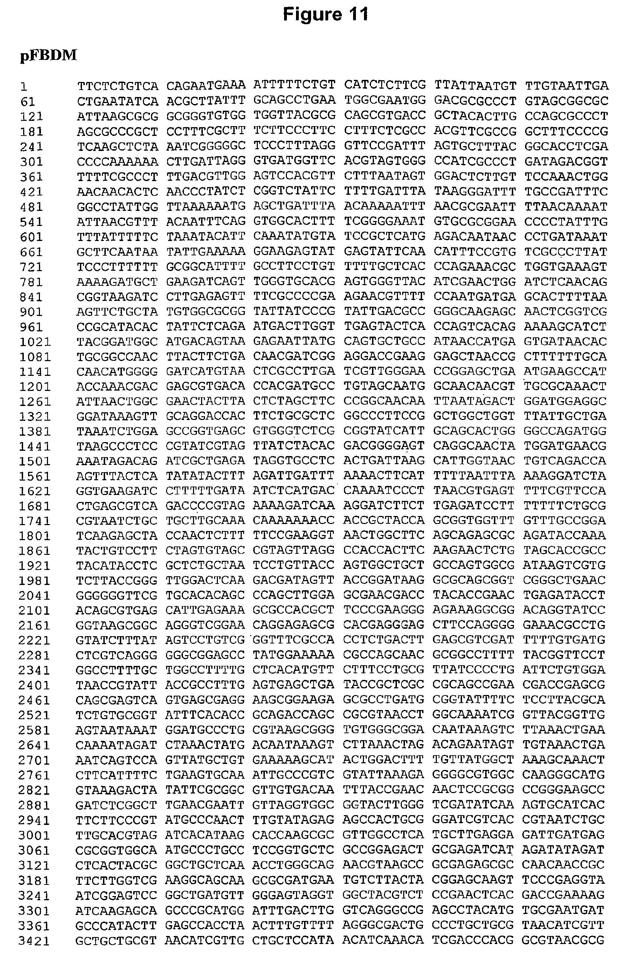

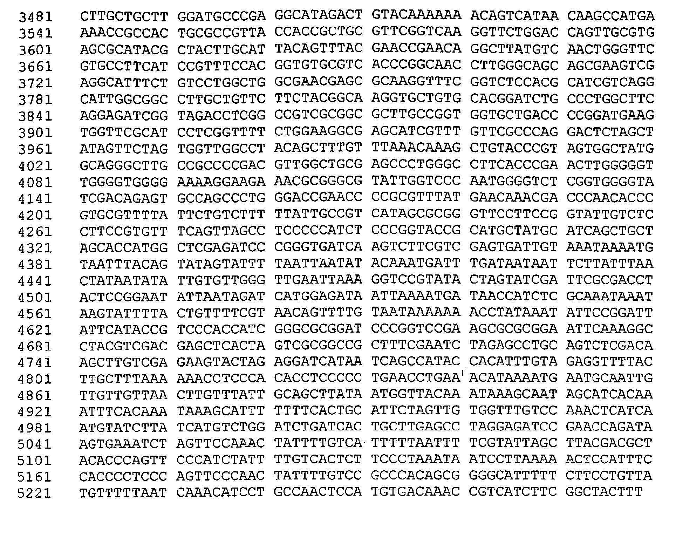

Figure 11 shows the complete polynucleotide sequence of a preferred embodiment of the present invention, the circular baculovirus transfer vector pFBDM.

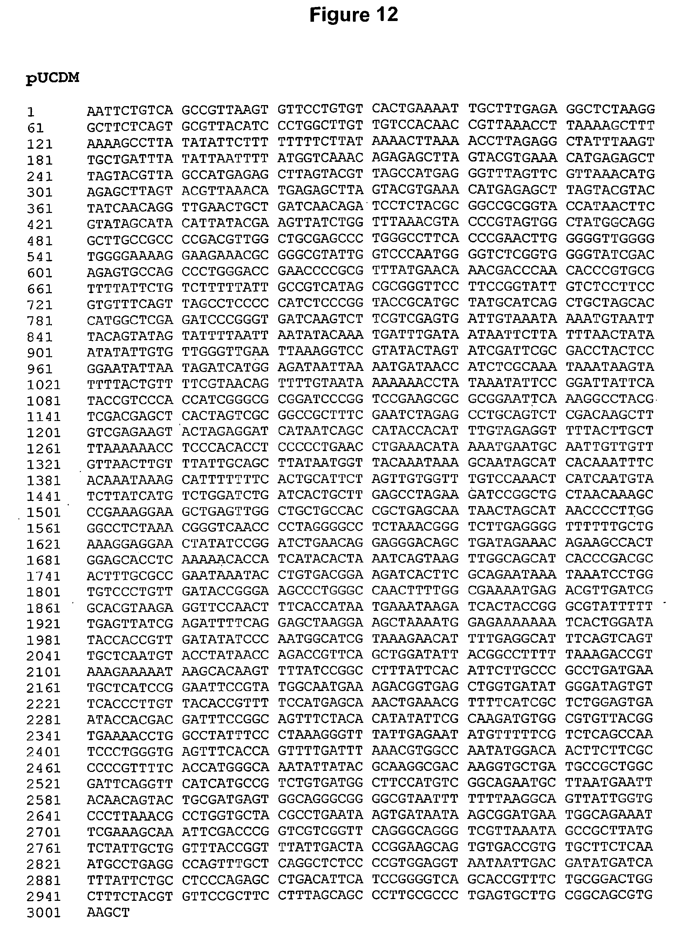

### Figure 12: Circular baculovirus transfer vector pUCDM

Figure 12 shows the complete polynucleotide sequence of a preferred embodiment of the present invention, the circular baculovirus transfer vector pUCDM.

### Fig. 13: In vivo screening for protein-protein interactions and modifications using MultiBac.

Figure 13 illustrates screening strategies using vectors of the present invention for assessing the physical association of proteins/protein complexes (I.) and/or modifications thereof (II.) *in vivo.*

In the following specific embodiments of the invention will be described by way of example and these examples are not to be construed to be limiting to the scope of the present invention.

### Examples

### I. New Baculovirus Tools for Multigene Applications

In the following new baculovirus transfer vectors according to the present invention constructed specifically for multigene applications are described in detail. These transfer vectors present a modified recipient baculovirus DNA engineered for improved protein production, and a simple and rapid method to integrate genes via two access sites (attTn7 and LoxP) into this baculoviral DNA in *E. coli* cells tailored for this purpose. Said vectors (pFBDM and pUCDM) contain a multiplication module and greatly facilitate modular combination of heterologous genes with a minimum requirement for unique restriction sites. Viral promoters (currently p10 and polh very late promoters) can be exchanged in these vectors to other promoter sequences (early, late) if required. Likewise, terminator sequences (currently SV40, HSVtk) can be substituted.

These vectors comprise an engineered baculovirus genome (MultiBac) with improved protein production properties. Two baculoviral genes were disrupted which leads to improved maintenance of cellular compartments during infection and protein production. The quality of proteins produced by this system is significantly improved through a reduction of viral dependent proteolytic activity and reduced cell lysis.

The vectors according to the invention allow for a new method for rapid combinatorial generation of recombinant baculovirus DNA by accessing the viral genome via two specific sites (see Fig. 1 for an overview). In addition to the commercially available Tn7 transposon site, a LoxP sequence was introduced at a separate site on the baculovirus genome which can accept multigene expression cassettes from the pUCDM plasmid by site-specific recombination. A protocol for carrying out Tn7 transposition (from pFBDM derivatives carrying multigene cassettes) and LoxP site-specific recombination (from pUCDM derivatives carrying multigene cassettes) efficiently in a single step in *E.coli* was developed. These protocols are useful not only to integrate multigene cassettes with coding sequences for multiprotein complex subunits into MultiBac, but also to integrate specific enzymes (kinases, acetylases etc.) for modifying the proteins under investigation.

### Example 1: Transfer vectors pFBDM and pUCDM and baculovirus vector MultiBac

The transfer vector pFBDM (SEQ ID NO:1, Fig. 11) contains two expression cassettes in a head-to-head arrangement with multiple cloning sites MCS1 and MCS2 flanked by polh or p10 promoters and SV40 or HSVtk polyA signal sequences, respectively. Multiplication module M is located in between the promoter sequences. The sequences used for Tn7 transposition (Tn7L and Tn7R) encompass the expression cassettes and a gentamycin resistance marker. For further details, see Fig. 2.

The transfer vector pUCDM (SEQ ID NO:2, Fig. 12) has an identical expression cassette including a multiplication module as pFBDM. This expression cassette is flanked by a LoxP inverted repeat. Vector pUCDM contains a chloramphenicol resistance marker and a conditional R6Kγ origin of replication which makes its propagation dependent on the expression of the *pir* gene in the prokaryotic host. For further details, see Fig. 3.

In short, both vectors were prepared as follows. Transfer vector pFBDM was derived from pFastBac^{™}DUAL (Invitrogen). The sequence 5'-TACTAGTATCGATTCGCG-ACC was inserted into the BstZ17I site between the polh and p10 promoters generating the multiplication module containing, in order, BstZ17I, Spel, Clal and Nrul cleavage sites. A site for the rare-cutter Pmel was added by replacing the SnaBI recognition sequence with the oligonucleotide 5'-AGCTTTGTTTAAACAAAGCT. Vector pUCDM was generated by combining the 998 bp EcoRV/AlwNI fragment of pUNI10 univector containing the R6Kγ origin with the 1258 bp AlwNI fragment from pLysS (Novagen) after treatment with mung bean nuclease (New England Biolabs). This pLysS fragment contains the chloramphenicol resistance marker. The resulting construct was digested with Smal and XbaI, and ligated to the 1016 bp PmeI/AvrII fragment of pFBDM. Unique restriction sites for AvrII and Pmel restriction endonucleases were reintroduced by site-directed mutagenesis using the QuickChange protocol (Stratagene). The vectors pUCDM and pFBDM contain identical expression cassettes including the multiplication module.

The baculoviral vector, termed MultiBac, which represents a bacmid as it contains an origin of replication (F-replicon) that allows its propagation in E.coli strains (e.g. DH10α and derivatives), was constructed in the following way. Plasmid pKlloxP, containing the linear fragment used to replace the *v-cath* and *chiA* genes in bMON14272 by ET recombination, was generated from pFastBac^{™}DUAL by recircularization of the 3385 bp StuI/FspI fragment thus eliminating these sites and disrupting the ampicillin resistance gene. This pKI vector has a gentamycin marker and the original pFastBac^{™}"DUAL p10 MCS containing Xhol, Ncol and Kpnl restriction sites. pKI was modified to yield pKIIoxP as follows. The 1008 bp BspHI fragment of pFastBac^{™}"DUAL containing the ampicillin resistance marker was introduced into the NcoI site. Next, homology region HomA from the baculovirus *chiA* gene was amplified from bMON14272 with primers 5'-GCGCGCCTCGAGGCC TCCCACGTGCCCGACCCCGGCCCG and 5'-GCGCGCCTCGAGGGAGGAGCT GCGCGCAATGC, and digested with Xhol. The resulting 408 bp fragment was inserted into the Xhol site of pKI. Homology region HomB from the *v-cath* gene was amplified with primers 5'-GCGCGCGGTACCGCGTTCGAAGCCATCATTA and 5'-GCGCGCGGTACCAGGCCTGAAAAATCCGTCCTCTCC, digested with Kpnl and the resulting 372 bp fragment inserted into the pKI KpnI site. Finally, a Cre-loxP sequence was introduced into the Nhel site by the oligonucleotide 5'-CTAGAATAACT TCGTATAGCATACATTATACGAAGTTAGTTTAAACT. The gene encoding for Cre recombinase and a six-histidine tag directly adjacent to the start codon was generated from synthetic oligonucleotides (Microsynth, CH) and ligated to NcoI/KpnI digested pBAD22 vector. The ampicillin resistance marker was inactivated by FspI/ScaI digestion. A Zeocin resistance gene under control of a synthetic prokaryotic promoter (EM7) was excised from pPICZA (Invitrogen) by PvuII/EcoRV digestion and ligated to the 5388-bp Fspl/Scal fragment yielding pBADZ-His₆Cre.

Transfer vector pFBDM, pKILoxP and pBADZ-His₆Cre are propagated in generic *E.Coli* strains such as TOP10 cells (Invitrogen), pUCDM is propagated in bacterial hosts BW23473 or BW23474 expressing the *pir* gene.

Next, for generation of baculoviral bacmid MultiBac, the vector pBAD-ETγ carrying truncated recE under the arabinose-inducible PBAD promoter and recT under the EM7 promoter was modified by placing the Zeocin resistance gene from pPICZA into the Fspl and Scal sites as described for pBADZ-His₆Cre yielding pBADZ-ETγ̃.This vector was transformed into DH10BAC^{™} (Invitrogen) cells harboring the bacmid bMON14272 and the helper plasmid pMON7124 yielding colonies resistant to kanamycin, tetracyclin and Zeocin. A single colony was used to inoculate 500 ml 2xTY (1.6% bacto tryptone, 1 % yeast extract, 0.5% NaCl) in the presence of these three antibiotics at 20 °C. At OD₆₀₀=0.25, L-arabinose (Fluka BioChemicals) was added to 0.1 % and the culture incubated until OD₆₀₀=0.5. Electro-competent DH10BacET cells were then generated following standard procedures and stored at - 80 °C. The vector pKIIoxP was transformed into JM110 cells (Stratagene) lacking *dam* and *dcm* activity. Unmethylated plasmid DNA was digested with Stul to yield a 1827 bp fragment containing the ampicillin resistance marker and a Cre-IoxP sequence flanked by *chiA* and *v-cath* homology regions. A minimum of 0.3 µg linear DNA was then electroporated into DH10BACET cells. Transformed cells were grown for 4 h at 37 °C and plated on agar plates containing kanamycin, tetracyclin and ampicillin. Bacmid DNA from two single triple-resistant colonies was analyzed by PCR amplification using primers 5'-AGGTACTAAATATGGCG and 5'-CTGAGCGACATCACT which anneal outside the homology regions and by sequencing the PCR fragment to confirm correct integration. Frozen electro-competent DH10MultiBac cells containing the modified bacmid having the *v-cath* and *chiA* genes replaced by an ampicillin marker and *LoxP* were prepared as above with ampicillin replacing Zeocin in the culture.

To generate composite MultiBac bacmids, pBADZ-His₆Cre was transformed into DH10MultiBac by electroporation for expression of Cre recombinase yielding clones resistant to kanamycin, tetracyclin and Zeocin. A single colony was used to inoculate 500 ml 2xTY in the presence of these antibiotics at 20 °C. L-arabinose was added to 0.1% at OD₆₀₀=0.25. At OD₆₀₀=0.5 frozen electro-competent DH10MultiBac^{Cre} cells containing overexpressed Cre recombinase were prepared. Plasmid pUCDM or derivatives were electroporated into DH10MultiBac^{Cre} cells and plated on agar containing kanamycin, tetracyclin and chloramphenicol. Triple resistant clones were assayed on plates containing IPTG and Bluogal for integrity of the *lacZ*α gene with *Tn7,* yielding blue colonies in all cases tested. Clones were analyzed for the correct Cre-loxP site-specific recombination event by PCR amplification and sequencing as described above. Loss of pBADZ-His₆Cre was confirmed by replating in the presence of Zeocin. Electro-competent cells were prepared from the individual clones in the presence of kanamycin, tetracyclin and chloramphenicol. Transposition of Tn7 elements from pFBDM derivatives into *Tn7*, identification of recombinants by blue/white screening, and transfection of purified composite bacmid was carried out according to established protocols (e.g. O'Reilly, D.R., Miller, L.K. & Luckow, V.A. "Baculovirus expression vectors. A laboratory manual." Oxford University Press, New York - Oxford (1994); "Bac-to-Bac^{™} Baculoviorus Expression Systems Manual." Invitrogen, Life Technologies Incorporated (2000)). For simultaneous transformation of DH10MultiBac^{Cre} with pUCDM and pFBDM derivatives, at least 1 µg of each was used.

A function of the F-replicon on the bacmid is to limit the copy-number to one or two, reducing the potential for undesired recombination. Introduction of pFBDM derivatives into MultiBac disrupts the *lacZ*α gene allowing unambiguous identification of cells containing only composite bacmid. However in the case of Cre-catalyzed fusion of pUCDM derivatives, co-existence of a copy of both the composite and parent bacmids can not be ruled out based on chloramphenicol resistance. Virus from initial transfections with MultiBac containing the EYFP gene inserted by Cre catalysis was therefore clonally separated by plaque purification. As 29 of 32 (91 %) plaque purified specimens expressed EYFP, Cre-IoxP site-specific recombination occurred with nearly saturating efficacy. Notwithstanding the presence of several copies of viral promoters (p10, polh) and terminators (SV40, HSVtk), MultiBac derivatives could be serially passaged at least five times in Sf21 cells at a multiplicity of infection (MOI) ≤ 1.

### Example 2: Generating multigene expression cassettes

The vectors pFBDM and pUCDM are particularly suited for generating multigene expression cassettes due to the multiplication module inserted in between the two promoters. The logic of multiplication is illustrated in Fig. 4. The only prerequisite for assembling multigene expression cassettes is that the restriction enzymes used for multiplication (e.g. PmeI, AvrII, SpeI, and either BstZ17I or Nrul) are unique, which can be easily accomplished for instance by site directed mutagenesis prior to multigene cassette assembly or provision of compounds (e.g. peptide nucleic acids) capable of masking additional sites that are not to be cleaved in the inserted encoding DNA sequences. Genes are cloned into MCS1 and MCS2 of pFBDM. The entire expression cassette is then excised by Pmel and AvrII digestion. The resulting fragment is placed into the multiplication module of a pFBDM derivative containing further sets of genes via either SpeI/BstZ17I or Spel/Nrul sites. Spel produces a cohesive end compatible with AvrII, while BstZ17I, Nrul and Pmel are blunt-cutters. The restriction sites involved are eliminated in the process and multiplication can be repeated iteratively using the module present in the inserted cassette. The same logic applies also for generation of pUCDM derivatives with multigene expression cassettes. Also, promoter and terminator sequences can be easily modified if desired using appropriate restriction sites in the vectors of the invention.

### Example 3: Baculovirus engineered for improved protein production

The baculovirus genome was modified to obtain improved protein production properties. Two baculoviral genes, *v-cath* and *chiA,* were disrupted which leads to improved maintenance of cellular compartments during infection and protein production. The *v-cath* gene encodes for a viral protease, V-CATH, which is activated upon cell death by a process dependent on a juxtaposed gene on the viral DNA, *chiA*, which encodes for a chitinase. Both genes were disrupted to eliminate V-CATH activity and to gain the option of utilizing chitin-affinity chromatography for purification without interference from the *chiA* gene product. The quality of proteins produced by this so-called MultiBac baculovirus is significantly improved through a reduction of viral-dependent proteolytic activity and reduced cell lysis. In place of the disrupted viral DNA sequence, a LoxP sequence for *cre-lox* site-specific recombination was placed. For further details see Fig. 5.

### Example 4: Cloning into pFBDM or pUCDM transfer vectors

The genes of choice are cloned into the multiple cloning sites MCS1 or MCS2 (see Fig. 2, 3) of pFBDM and pUCDM using standard cloning procedures. Ligation reactions for pFBDM derivatives are transformed into standard *E. coli* cells for cloning (such as TOP10, DH5α, HB101) and plated on agar containing ampicillin (100 µg/ml). Ligation reactions for pUCDM derivatives are transformed into *E. coli* cells expressing the *pir* gene (such as BW23473, BW23474) and plated on agar containing chloramphenicol (25 µg/ml). Correct clones are selected based on specific restriction digestion and DNA sequencing of the inserts.

### Example 5: Cre-Iox site-specific recombination protocol

Approximately 5-10 ng of the pUCDM derivative are incubated on ice (15 min) with 50-100 µl electro-competent DH10MultiBac^{Cre} cells. Following electroporation (200 ohms, 25 µF, 1.8 kV pulse), cells are incubated at 37 °C for 8 hours and plated on agar containing kanamycin (50 µg/ml), chloramphenicol (25 µg/ml) and ampicillin (100 µg/ml). Colonies appear after incubation at 37 °C (12-15 hours). Then bacmids can be prepared for insect cell transfection (see III.6.) or clones can be prepared for integration of a pFBDM derivative by Tn7 transposition (see below).

### Example 6: Transposition protocol for pFBDM derivatives

Approximately 5-10 ng of the pFBDM derivative are incubated on ice (15 min) with 50-100 µl electro-competent DH10MultiBac^{Cre} cells. Following electroporation (200 ohms, 25 µF, 1.8 kV pulse), cells are incubated at 37 °C for 8 hours and plated on agar containing kanamycin (50 µg/ml), gentamycin (7 µg/ml), ampicillin (100 µg/ml), tetracyclin (10 µg/ml), BluoGal (100 µg/ml) and IPTG (40 µg/ml). White colonies are selected after incubation at 37 °C (18 - 24 hours). Then bacmids can be prepared for insect cell infection (see below).

### Example 7: One-step transposition / cre-lox site-specific recombination

*Cre-Iox* site-specific recombination and Tn7 transposition can be carried out simultaneously in DH10MultiBac^{Cre} cells, if desired. Since the efficacy of a double transformation is reduced as compared to transformation with one plasmid only, significantly larger amounts of DNA have to be utilized in this reaction. Approximately 2-4 µg of the pFBDM derivative and 2-4 µg of the pUCDM derivative of choice are incubated on ice (15 min) with 50-100 µl electro-competent DH10MultiBac^{Cre} cells. Following electroporation (200 ohms, 25 µF, 1.8 kV pulse) cells are incubated at 37 °C for 8 hours and plated on agar containing chloramphenicol (25 µg/ml), kanamycin (50 µg/ml), gentamycin (7 µg/ml), tetracyclin (10 µg/ml), BluoGal (100 µg/ml) and IPTG (40 µg/ml). White colonies are selected after incubation at 37 °C (18 - 24 hours). Next, bacmids can be prepared for insect cell infection (see below).

### Example 8: Preparation of cre-lox integrands for Tn7 transposition

For certain applications, it can be advantageous to add protein genes for expression to a MultiBac baculoviral DNA which already carries a set of foreign genes integrated by Cre catalysis. Then, the DH10MultiBac^{Cre} cells harboring recombinant MultiBac with an integrated pUCDM derivative (see III.2.) are restreaked on agar containing chloramphenicol (25 µg/ml), kanamycin (50 µg/ml), ampicillin (100 µg/ml), tetracyclin (10 µg/ml), BluoGal (100 µg/ml) and IPTG (40 µg/ml). A blue colony with a transposition-competent MultiBac derivative is then incubated in 500 ml 2xTY medium containing chloramphenicol (25 µg/ml), kanamycin (50 µg/ml), ampicillin (50 µg/ml), and tetracyclin (10 µg/ml) at 37 °C until OD₆₀₀ reaches 0.5. The culture is then cooled on ice (15 min), and centrifuged (4000 rpm, 8 min). The cell pellet is resuspended in 250 ml ice-cold 10% glycerol solution (sterile) and centrifuged (4000 rpm, 8 min). The cell pellet is then resuspended in 200 ml ice-cold 10% glycerol solution (sterile) and centrifuged again (4000 rpm, 8 min). Then, the cell pellet is resuspended in 50 ml ice-cold 10% glycerol solution (sterile), and finally after centrifugation in 1 ml 10% glycerol solution (sterile). Cells are frozen in 50-100 µl aliquots in liquid nitrogen and stored at -80 °C. Transposition of pFBDM derivatives can be carried out as described below.

### Example 9: Bacmid preparation and infection of insect cells

Preparation of bacmid DNA, infection of insect cells and protein expression is carried out according to established protocols (e.g. O'Reilly, D.R., Miller, L.K. & Luckow, V.A. "Baculovirus expression vectors. A laboratory manual." Oxford University Press, New York - Oxford (1994); "Bac-to-Bac^{™} Baculoviorus Expression Systems Manual." Invitrogen, Life Technologies Incorporated, (2000)).

### Example 10: Efficiency of cre-lox site-specific recombination into MultiBac

The propagation of MultiBac in DH10MultiBac^{Cre} cells is dependent on the presence of the F-replicon on the bacmid. A function of the F-replicon is the tight control of the copy-number (one or two), reducing the potential for undesired recombination. Introduction of pFBDM derivatives into MultiBac disrupts the *lacZ*α gene thus allowing for unambiguous identification of cells containing only composite bacmid. In case of Cre-catalyzed integration of pUCDM derivatives, however, a co-existence of one composite bacmid and one parent MultiBac molecule can not be ruled out based on chloramphenicol resistance. Virus from initial transfections with MultiBac containing a gene for yellow fluorescent protein EYFP inserted by Cre catalysis was therefore clonally separated by plaque purification. 29 of 32 (91 %) plaque purified specimens expressed EYFP, arguing for a *cre-lox* site-specific recombination reaction with close to saturating efficacy. (for further detail see Fig. 6)

### Example 11: Effect of chiA and v-cath deletion in MultiBac

During heterologous protein production using a commercially available baculovirus expression system, viral-dependent proteolytic breakdown consistent with the action of a cysteine protease was observed. The baculoviral *v-cath* gene encodes for a cysteine protease which is directly involved in liquefaction of the insect cell host. V-CATH is activated upon cell death by a process dependent on a juxtaposed gene on the viral DNA, *chiA*, which encodes for a chitinase. Disruption of both genes eliminated V-CATH activity and further provided the option of utilizing chitin-affinity chromatography for purification without interference from the *chiA* gene product. The effect of the disruption was assayed by comparing samples from cells infected with MultiBac virus to cells infected with a commercially available baculovirus carrying *v-cath* and *chiA*. A strongly reduced proteolytic activity, concomitant with much improved maintenance of cell integrity, was observed in lysate from cells infected with MultiBac. (for more detail, see Fig. 7).

### Example 12: Production of a 275 kD protein complex using MultiBac

In a test experiment the yeast lsw2 chromatin remodelling complex consisting of the 130 kDa Isw2p and the 145 kDa Itc1p proteins was expressed. One version of MultiBac was created with both proteins integrated at the attTn7 site, and a second version with lsw2p integrated at the attTn7 site and Itc1p inserted at the LoxP site. Spatial decoupling of the two genes on the virus did not adversely affect either relative production levels of the subunits or functional assembly of the lsw2 complex in insect cells, exemplifying the utility of both Cre-Iox and Tn7 sites for simultaneous integration and expression of proteins. For further detail see Fig. 8.

### Example 13: Production of multiprotein complexes using MultiBac

In addition to the yeast lsw2 remodeling complex also the three membered yeast Isw1b complex (360 kD) and a human transcription factor complex (700 kDa) were expressed using MultiBac. Further, in all cases also EYFP fluorescent protein was co-expressed. The constant yield of EYFP and the respective complexes in the co-expression experiments illustrated that expression is not saturating in this system and suggests that even much larger complexes containing many more subunits can be expressed using MultiBac. For further detail see Fig. 9.

### Example 14: MultiBac for gene transfer in mammalian cells ("BacMam")

A MultiBac derivative virus containing flurescent proteins EYFP and ECFP under control of baculoviral promoters and dsRED under control of CMV late promoter were generated. Insect cells infected with this hybrid virus showed expression of EYFP and ECFP. Mammalian COS cells infected with virus amplified in insect cells showed strong fluorescence of dsRED protein, demonstrating the potential use of MultiBac for multigene transfer into mammalian cells ("BacMam"). For further detail see Fig. 10

### II The MultiBac Systems Kit

A MultiBac system kit as disclosed herein may comprise one or more of the following:
DH10MultiBac^{Cre} cells, BW23474, BW23474 cells^{†}, pFBDM vector, pUCDM vector, control plasmid for Tn7 transposition*, control plasmid for *cre-lox* site-specific recombination*, a generic transfectant reagent^{#}
^{†} *E. coli* strains expression the *pir* gene for propagation of pUCDM derivatives (any other strain with *pir*+ background can be used).
* In the experiments, vectors carrying genes for fluorescent proteins ECFP and EYFP were used as controls. These genes are marketed under license by Molecular Probes. They are particularly useful as controls since the observation of fluorescence either by fluorescent microscopy or by using a fluorescence spectrophotometer is entirely straight forward. However, any type of control plasmid carrying a gene encoding for a protein that can be identified with ease (glucurodinase, catechol dioxygenase, XylE, luciferase etc.) can be utilized.
^{#} For example CellFECTIN (Invitrogen), LipoTAXI® Transfection Reagent (Stratagene) etc.

## Claims

1. A polynucleotide for multigene applications comprising a functional arrangement according to Fig. 1 said arrangement comprising
(a) at least two expression cassettes T1 - MCS1 - P1 and P2 - MCS2 - T2 in a head to head, head-to-tail or tail-to-tail arrangement, each comprising a multiple cloning site MCS1 or MCS2. flanked by a promoter P1 and a terminator sequence T1 for MCS1 and flanked by a promoter P2 and a terminator sequence T2 for MCS2,
(b) at least one multiplication module M in between the promoters P1 and P2 comprising at least 2 restriction sites A and B,
(c) at least two restriction sites X and Y each flanking one of the expression cassettes,
wherein:
(i) restriction sites A and X as well as B and Y are compatible, but
(ii) the ligation products of AY and BX are not enzymatically cleaved by restriction enzymes a, b, x or y, specific for restriction sites A, B, X, and Y, and
(iii) restriction sites A and B as well as X and Y are not compatible.

2. The polynucleotide according to claim 1, wherein the restriction sites A and B in the multiplication module M are selected from the group consisting of BstZ171, Spel, Clal and Nrul or restriction enzymes that have identical cleavage sites as said enzymes ("isoschizomers").

3. The polynucleotide according to claim 1 or 2, wherein the restriction sites X and Y are selected from the group consisting of Pmel and AvrII or restriction enzymes that have identical cleavage sites as said enzymes ("isoschizomers").

4. The polynucleotide according to any one of claims 1 to 3, wherein the promoters P1 and P2 are selected from the group consisting of polh, p10 and P_{XIV} very late baculoviral promoters, vp39 baculoviral late promoter, vp39polh baculoviral late/very late hybrid promoter P_{cap/polh}, *pcna*, *etl*, *p35*, *egt, da26* baculoviral early promoters; CMV, SV40, UbC, EF-1α, RSVLTR, MT, P_{DS47}, Ac5, P_{GAL} and P_{ADH}.

5. The polynucleotide according to any one of claims 1 to 4, wherein the terminator sequences T1 and T2 are selected from SV40, HSVtk or BGH (bovine growth hormone).

6. The polynucleotide according to any one of claims 1 to 5, additionally comprising at least one site for its integration into a vector or host cell.

7. The polynucleotide according to claim 6, wherein the integration site is selected from the group consisting of the transposon elements of Tn7, λ-integrase specific attachment sites and SSRs (site specific recombinases), preferably the cre-lox specific recombination (LoxP) site or the FLP recombinase specific recombination (FRT) site.

8. The polynucleotide according to any one of claims 1 to 7, wherein said polynucleotide comprises
(a) promoters P1 and P2 selected from polh and p10,
(b) terminator sequences selected from SV40 and HSVtk.
(c) restriction sites A and B in the multiplication module M selected from the group consisting of BstZ171, SpeI, ClaI and NruI,
(d) restriction sites X and Y selected from the group consisting of Pmel and AvrII,
(d) sites for virus integration selected from the cre-lox and Tn7 recombination system.

9. A vector comprising a polynucletide sequence according to any one of claims 1 to 8.

10. The vector of claim 9, wherein said vector is a virus selected from the group consisting of adenovirus, adeno-associated virus (AAV), autonomous parvovirus, herpes simplex virus (HSV), retrovirus, rhadinovirus. Epstein-Barr virus, lentivirus, semliki forest virus and baculovirus.

11. The vector of claim 10, wherein the vector is a baculovirus expression vector.

12. The vector according to claim 11, wherein two baculoviral genes v-cath and chiA are functionally disrupted.

13. The vector according to claims 9 to 12, additionally comprising a site for SSRs (site specific recombinases), preferably LoxP for cre-lox site specific recombination.

14. The vector according to claim 12 and 13, wherein the cre-lox site is located in one or both of the two baculoviral genes v-cath and chiA.

15. The vector according to any one of claims 9 to 14, comprising a transposon element, preferably the Tn7 attachment site.

16. The vector of claim 9, wherein said vector has the sequence according to SEQ ID NO: 1 (pFBDM).

17. The vector of claim 9, wherein said vector has the sequence according to SEQ ID NO: 2 (pUCDM).

18. A host cen comprising a polynucleotide according to any one of claims 1 to 8 and/or a vector according to any one of claims 9 to 17.

19. The host cell according to claim 18, selected from the group consisting of mammalian cells, preferably human cells, rodent cells, porcine cells, bovine cells, ovine cells, *and C. elegans* cells; yeast cells, preferably *S. cervisiae*, *S.pombe, C. albicans* and *P. pastoris*; insect cells; and *E*. *coli* cells.

20. A recombinant non-human animal comprising a polynucleotide according to any one of claims 1 to 8 and/or a vector according to any one of claims 9 to 17.

21. The non-human animal of claim 20, wherein the animal is selected from mammals, preferably, rodent, porcine and bovine, *and C.elegans*.

22. A method for generating multigene expression cassettes, comprising the steps of
(a) cloning one or more genes into MCS1 and/or MCS2 of a first vector according to any one of claims 9 to 17,
(b) cloning one or more genes into MCS1 and/or MCS2 of a second vector according to any one of claims 9 to 17,
(c) excising the entire functional arrangement at the restriction sites X and Y in the first transfer vector,
(d) cutting the second transfer vector at the multiplication module M,
(e) ligating the excised functional arrangement of step (c) into the cut multiplication site of the second vector of step (d), thereby producing a third transfer vector having the multiplication module of the first transfer vector and expression cassettes of both the first and the second vector, and
(f) optionally repeating steps (a) through (e) for assembling a transfer vector with multiple expression cassettes.

23. A method for producing multiprotein complexes *in vitro* comprising the steps of
(a) generating multigene expression cassettes according to claim 22,
(b) transferring the multigene expression cassettes into a host cell capable of expressing said genes simultaneously.

## Patentansprüche

1. Ein Polynukleotid für Multigenanwendungen umfassend eine funktionelle Anordnung gemäss Fig. I wobei die Anordnung das Folgende umfasst
(a) wenigstens zwei Expressionskassetten T1 - MCS1 - P1 und P2 - MCS2 - T2 in einer Kopf-zu-Kopf-, Kopf-zu-Schwanz- oder Schwanz-zu-Schwanz-Anordnung, jeweils umfassend eine Mehrfachklonierungsstelle MCS1 oder MCS2, flankiert von einem Promoter P1 und einer Terminationssequenz T1 für MCS1 und flankiert von einem Promoter P2 und einer Terminationssequenz T2 für MCS2,
(b) wenigstens ein Multiplikationsmodul M zwischen den Promoteren P1 und P2 umfassend wenigstens 2 Restriktionsschnittstellen A und B,
(c) wenigstens zwei Restriktionsschnittstellen X und Y, die jeweils eine der Expressionskassetten flankieren,
wobei:
(i) Restriktionsstellen A und X sowie B und Y kompatibel sind, aber
(ii) die Ligationsprodukte von AY und BX nicht durch die Restriktionsenzyme a, b, x oder y enzymatisch gespalten werden, die für die Restriktionsschnittstellen A, B, X, und Y spezifisch sind, und
(iii) die Restriktionsschnittstellen A und B sowie X und Y nicht kompatibel sind.

2. Das Polynukleotid gemäss Anspruch 1, wobei die Restriktionsschnittstellen A und B in dem Multiplikationsmodul M aus der Gruppe ausgewählt sind, bestehend aus BstZ171, SpeI, ClaI und Nrul oder Restriktionsenzymen, die zu den genannten Enzymen identische Spaltstellen aufweisen ("Isoschizomere").

3. Das Polynukleotid gemäss Anspruch 1 oder 2, wobei die Restriktionsschnittstellen X und Y aus der Gruppe ausgewählt sind, bestehend aus Pmel und AvrII oder Restriktionsenzymen, die zu den genannten Enzymen identische Spaltstellen aufweisen ("Isoschizomere").

4. Das Polynukleotid gemäss einem der Ansprüche 1 bis 3, wobei die Promotoren P1 und P2 aus der Gruppe ausgewählt sind, bestehend aus polh, p10 und p_{XIV} sehr späten Baculoviruspromoteren, vp39 spätem Baculoviruspromoter, vp39polh spätem/sehr spätem Baculovirushybridpromoter P_{cap/polh}, *pcna, etl, p35, egt, da26* frühen Baculoviruspromotoren; CMV, SV40, UbC, EF-1α, RSVLTR, MT, P_{DS47}, Ac5, P_{GAL} and P_{ADH}.

5. Das Polynukleotid gemäss einem der Ansprüche 1 bis 4, wobei die Terminationssequenzen T1 and T2 aus SV40, HSVtk oder BGH (bovine growth hormone, Rinderwachstumshormon) ausgewählt sind.

6. Das Polynukleotid gemäss einem der Ansprüche 1 bis 5, das zusätzlich wenigstens eine Stelle für dessen Integration in einen Vektor oder eine Wirtszelle umfasst.

7. Das Polynukleotid gemäss Anspruch 6, wobei die Integrationsstelle aus der Gruppe ausgewählt ist, bestehend aus den Transposonelementen von Tn7, λintegrase-spezifischen Anheftungsstellen und SSRs (site specific recombinases, positionsspezifischen Rekombinasen), vorzugsweise der cre-loxspezifischen Rekombinationsstelle (LoxP) oder der FLP-recombinasespezifischen Rekombinationsstelle (FRT).

8. Das Polynukleotid gemäss einem der Ansprüche 1 bis 7, wobei das Nukleotid das Folgende umfasst
(a) Promoteren P1 und P2 ausgewählt aus polh und p10,
(b) Terminationssequenzen ausgewählt aus SV40 und HSVtk,
(c) Restriktionsschnittstellen A und B in dem Multiplikationsmodul M ausgewählt aus der Gruppe bestehend aus BstZ171, SpeI, Clal und Nrul,
(d) Restriktionsschnittstellen X und Y ausgewählt aus der Gruppe bestehend aus PmeI und AvrII,
(d) Stellen zur Virusintegration ausgewählt aus dem cre-lox- und Tn7-Rekombinationssystem.

9. Ein Vektor umfassend eine Polynucletidsequenz gemäss einem der Ansprüche 1 to 8.

10. Der Vektor von Anspruch 9, wobei der Vektor ein Virus ausgewählt aus der Gruppe bestehend aus Adenovirus, adenoassoziiertem Virus (AAV), autonomer Parvovirus, Herpes Simplex-Virus (HSV), Retrovirus, Rhadinovirus, Epstein-Barr-Virus, Lentivirus, Semliki-Forest-Virus and Baculovirus ist.

11. Der Vektor von Anspruch 10, wobei der Vektor ein Baculovirusexpressionsvektor ist.

12. Der Vektor gemäss Anspruch 11, wobei zwei Baculovirusgene v-cath und chiA funktionell zerstört sind.

13. Der Vektor gemäss den Ansprüchen 9 bis 12, der zusätzlich eine Stelle für SSRs (site specific recombinases, positionsspezifische Rekombinasen), vorzugsweise LoxP für die cre-lox-positionsspezifische Rekombination umfasst.

14. Der Vektor gemäss Anspruch 12 und 13, wobei die cre-lox-Stelle in einem oder beiden der zwei Baculovirusgene v-cath und chiA liegt.

15. Der Vektor gemäss einem der Ansprüche 9 bis 14, der ein Transposonelement, vorzugsweise die Tn7-Anheftungsstelle umfasst.

16. Der Vektor von Anspruch 9, wobei der Vektor die Sequenz gemäss SEQ ID NO: 1 (pFBDM) aufweist.

17. Der Vector von Anspruch 9, wobei der Vektor die Sequenz gemäss SEQ ID NO: 2 (pUCDM) aufweist.

18. Eine Wirtszelle, die ein Polynukleotid gemäss einem der Ansprüche 1 to 8 und/oder einen Vektor gemäss einem der Ansprüche 9 bis 17 umfasst.

19. Die Wirtszelle gemäss Anspruch 18 ausgewählt aus der Gruppe bestehend aus Säugetierzellen, vorzugsweise menschlichen Zellen, Nagerzellen, Schweinezellen, Rinderzellen, Schafszellen und *C. elegans-Zellen;* Hefezellen, vorzugsweise *S. cerevisiae, S.pombe, C. albicans* und *P. pastoris*; Insektenzellen; und *E. coli*-Zellen.

20. Ein rekombinantes, nicht-menschliches Tier, das ein Polynukleotid gemäss einem der Ansprüche 1 bis 8 und/oder einen Vektor gemäss einem der Ansprüche 9 bis 17 umfasst.

21. Das nicht-menschliche Tier von Anspruch 20, wobei das Tier aus Säugetieren, vorzugsweise Nager, Schwein und Rind sowie *C. elegans* ausgewählt ist.

22. Ein Verfahren zur Herstellung von Multigenexpressionskassetten umfassend die folgenden Schritte
(a) Klonieren eines oder mehrerer Gene in MCS1 und/oder MCS2 eines ersten Vektors gemäss einem der Ansprüche 9 bis 17,
(b) Klonieren eines oder mehrerer Gene in MCS1 und/oder MCS2 eines zweiten Vektors gemäss einem der Ansprüche 9 bis 17,
(c) Herausschneiden der gesamten funktionellen Anordnung an den Restriktionsschnittstellen X und Y in dem ersten Transfervektor,
(d) Schneiden des zweiten Transfervektors an dem Multiplikationsmodul M,
(e) Ligieren der herausgeschnittenen funktionellen Anordnung von Schritt (c) in die geschnittene Multiplikationsstelle des zweiten Vektors von Schritt (d), wodurch ein dritter Transfervektor mit dem Multiplikationsmodul des ersten Transfervektors und Expressionskassetten von beiden, dem ersten und dem zweiten Vektor hergestellt wird, und
(f) wahlweise das Wiederholen der Schritte (a) bis (e) zum Zusammensetzen eines Transfervektors mit mehreren Expressionskassetten.

23. Ein Verfahren zur Herstellung von Multiproteinkomplexen *in vitro* umfassend die Schritte des
(a) Herstellens von Multigenexpressionskassetten gemäss Anspruch 22,
(b) Transferierens der Multigenexpressionskassetten in eine Wirtszelle, die in der Lage ist, die Gene gleichzeitig zu exprimieren.

## Revendications

1. Polynucléotide pour des applications de multigènes comprenant un arrangement fonctionnel selon la Fig. I: ledit arrangement comprenant:
(a) au moins deux cassettes d'expression T1 - MCS1 - P1 et P2 - MCS2 - T2 dans un arrangement de tête à tête, de tête à queue ou de queue à queue, chacune comprenant un site de clonage multiple MCS1 ou MCS2, flanqué par un promoteur P1 et une séquence de terminaison T1 pour MCS1 et flanqué par un promoteur P2 et une séquence de terminaison T2 pour MCS2,
(b) au moins un module de multiplication M intermédiaire des promoteurs P1 et P2 comprenant au moins 2 sites de restriction A et B,
(c) au moins deux sites de restriction X et Y, flanquant chacun une des cassettes d'expression,
dans lequel:
(i) les sites de restriction A et X aussi bien que B et Y sont compatibles, mais
(ii) les produits de ligature de AY et de BX ne sont pas clivés enzymatiquement par les enzymes de restriction a, b, x ou y, spécifiques pour les sites de restriction A, B, X et Y, et
(iii) les sites de restriction A et B aussi bien que X et Y ne sont pas compatibles.

2. Polynucléotide selon la revendication 1, dans lequel les sites de restriction A et B dans le module de multiplication M sont choisis dans le groupe constitué de BstZ171, SpeI, ClaI et NruI ou des enzymes de restriction qui possèdent des sites de clivage identiques à ceux desdites enzymes (« isoschizomères »).

3. Polynucléotide selon la revendication 1 ou 2, dans lequel les sites de restriction X et Y sont choisis dans le groupe constitué de PmeI et AvrII ou des enzymes de restriction qui possèdent des sites de clivage identiques à ceux desdites enzymes (« isoschizomères »).

4. Polynucléotide selon l'une quelconque des revendications 1 à 3, dans lequel les promoteurs P1 et P2 sont choisis dans le groupe constitué des promoteurs très tardifs de baculovirus polh, p10 et p_{XIV}, du promoteur tardif de baculovirus vp39, du promoteur hybride tardif/très tardif de baculovirus vp39polh, des promoteurs précoces de baculovirus P_{cap/polh}, *pcna, etl, p35, egt, da26*; CMV, SV40, UbC, EF-1α, RSVLTR, MT, P_{DS47}, Ac5, P_{GAL} et P_{ADH}.

5. Polynucléotide selon l'une quelconque des revendications 1 à 4, dans lequel les séquences de terminaison T1 et T2 sont choisies parmi SV40, HSVtk ou BGH (hormone de croissance bovine).

6. Polynucléotide selon l'une quelconque des revendications 1 à 5, comprenant en outre au moins un site pour son intégration dans un vecteur ou une cellule hôte.

7. Polynucléotide selon la revendication 6, dans lequel le site d'intégration est choisi dans le groupe constitué des éléments de transposon de Tn7, des sites de fixation spécifiques à la λ-intégrase et des SSRs (recombinases spécifiques à un site), de préférence le site de recombinaison spécifique au cre-lox (LoxP) ou le site de recombinaison spécifique à la recombinase FLP (FRT).

8. Polynucléotide selon l'une quelconque des revendications 1 à 7, où ledit polynucléotide comprend:
(a) les promoteurs P1 et P2 choisis parmi polh et p10,
(b) les séquences de terminaison choisies parmi SV40 et HSVtk,
(c) les sites de restriction A et B dans le module de multiplication M choisis dans le groupe constitué de BstZ171, SpeI, ClaI et NruI,
(d) les sites de restriction X et Y choisis dans le groupe constitué de PmeI et AvrII,
(e) les sites pour une intégration de virus choisis parmi le système de recombinaison de cre-lox et de Tn7.

9. Vecteur comprenant une séquence de polynucléotide selon l'une quelconque des revendications 1 à 8.

10. Vecteur selon la revendication 9, dans lequel ledit vecteur est un virus choisi dans le groupe constitué d'adénovirus, de virus associé adéno (AVV), de parvovirus autonome, de virus de l'herpès simplex (HSV), de rétrovirus, de rhadinovirus, de virus de Epstein-Barr, de lentivirus, de virus de semliki forest et de baculovirus.

11. Vecteur selon la revendication 10, où le vecteur est un vecteur d'expression de baculovirus.

12. Vecteur selon la revendication 11, dans lequel deux gènes de baculovirus v-cath et chiA sont rompus fonctionnellement.

13. Vecteur selon les revendications 9 à 12, comprenant en outre un site pour des SSRs (recombinases spécifiques à un site), de préférence LoxP pour une recombinaison spécifique au site cre-lox.

14. Vecteur selon les revendications 12 et 13, dans lequel le site cre-lox est localisé dans un ou les deux gènes de baculovirus v-cath et chiA.

15. Vecteur selon l'une quelconque des revendications 9 à 14, comprenant un élément de transposon, de préférence le site de fixation de Tn7.

16. Vecteur selon la revendication 9, où ledit vecteur possède la séquence selon la SEQ ID NO: 1 (pFBDM).

17. Vecteur selon la revendication 9, où ledit vecteur possède la séquence selon la SEQ ID NO: 2 (pUCDM).

18. Cellule hôte comprenant un polynucléotide selon l'une quelconque des revendications 1 à 8 et/ou un vecteur selon l'une quelconque des revendications 9 à 17.

19. Cellule hôte selon la revendication 18, choisie dans le groupe constitué de cellules mammifères, de préférence des cellules humaines, des cellules de rongeurs, des cellules porcines, des cellules bovines, des cellules ovines et des cellules de *C. elegans*; des cellules de levure, de préférence *S. cervisiae, S. pombe, C. albicans* et *P. pastoris*; des cellules d'insectes; et des cellules de *E. coli.*

20. Animal non humain recombinant comprenant un polynucléotide selon l'une quelconque des revendications 1 à 8 et/ou un vecteur selon l'une quelconque des revendications 9 à 17.

21. Animal non humain selon la revendication 20, où l'animal est choisi parmi des mammifères, de préférence un rongeur, un porc et un bovin, et *C. elegans*.

22. Procédé pour la génération de cassettes d'expression de multigènes, comprenant les étapes:
(a) de clonage d'un ou de plusieurs gènes dans MCS1 et/ou MCS2 d'un premier vecteur selon l'une quelconque des revendications 9 à 17,
(b) de clonage d'un ou de plusieurs gènes dans MCS1 et/ou MCS2 d'un deuxième vecteur selon l'une quelconque des revendications 9 à 17,
(c) d'excision de l'arrangement fonctionnel entier aux sites de restriction X et Y dans le premier vecteur de transfert,
(d) de coupe du deuxième vecteur de transfert au module de multiplication M,
(e) de ligature de l'arrangement fonctionnel excisé de l'étape (c) dans le site de multiplication coupé du deuxième vecteur de l'étape (d), produisant ainsi un troisième vecteur de transfert possédant le module de multiplication du premier vecteur de transfert et les cassettes d'expression à la fois du premier et du deuxième vecteur, et
(f) éventuellement de répétition des étapes (a) à (e) pour l'assemblage d'un vecteur de transfert avec des cassettes d'expression multiples.

23. Procédé pour la production de complexes multiprotéiniques in vitro comprenant les étapes:
(a) de génération de cassettes d'expression de multigènes selon la revendication 22,
(b) de transfert des cassettes d'expression de multigènes dans une cellule hôte capable d'exprimer lesdits gènes simultanément.


### SEQUENCE LISTING

<110> Eidgenoessische Technische Hochschule zurich
<120> New expression tools for multiprotein applications
<130> RP20504
<140>
   <141> 2004-11-25
<150> US 60/552,072
   <151> 2004-03-09
<160> 2
<170> PatentIn version 3.2
<210> 1
   <211> 5279
   <212> DNA
   <213> baculovirus
<220>
   <221> misc_feature
   <223> Description of sequence: baculovirus vector pFBDM
<400> 1
   ttctctgtca cagaatgaaa atttttctgt catctcttcg ttattaatgt ttgtaattga      60
   ctgaatatca acgcttattt gcagcctgaa tggcgaatgg gacgcgccct gtagcggcgc     120
   attaagcgcg gcgggtgtgg tggttacgcg cagcgtgacc gctacacttg ccagcgccct     180
   agcgcccgct cctttcgctt tcttcccttc ctttctcgcc acgttcgccg gctttccccg     240
   tcaagctcta aatcgggggc tccctttagg gttccgattt agtgctttac ggcacctcga     300
   ccccaaaaaa cttgattagg gtgatggttc acgtagtggg ccatcgccct gatagacggt     360
   ttttcgccct ttgacgttgg agtccacgtt ctttaatagt ggactcttgt tccaaactgg     420
   aacaacactc aaccctatct cggtctattc ttttgattta taagggattt tgccgatttc     480
   ggcctattgg ttaaaaaatg agctgattta acaaaaattt aacgcgaatt ttaacaaaat     540
   attaacgttt acaatttcag gtggcacttt tcggggaaat gtgcgcggaa cccctatttg     600
   tttatttttc taaatacatt caaatatgta tccgctcatg agacaataac cctgataaat     660
   gcttcaataa tattgaaaaa ggaagagtat gagtattcaa catttccgtg tcgcccttat     720
   tccctttttt gcggcatttt gccttcctgt ttttgctcac ccagaaacgc tggtgaaagt     780
   aaaagatgct gaagatcagt tgggtgcacg agtgggttac atcgaactgg atctcaacag     840
   cggtaagatc cttgagagtt ttcgccccga agaacgtttt ccaatgatga gcacttttaa     900
   agttctgcta tgtggcgcgg tattatcccg tattgacgcc gggcaagagc aactcggtcg     960
   ccgcatacac tattctcaga atgacttggt tgagtactca ccagtcacag aaaagcatct    1020
   tacggatggc atgacagtaa gagaattatg cagtgctgcc ataaccatga gtgataacac    1080
   tgcggccaac ttacttctga caacgatcgg aggaccgaag gagctaaccg cttttttgca    1140
   caacatgggg gatcatgtaa ctcgccttga tcgttgggaa ccggagctga atgaagccat    1200
   accaaacgac gagcgtgaca ccacgatgcc tgtagcaatg gcaacaacgt tgcgcaaact 1260
   attaactggc gaactactta ctctagcttc ccggcaacaa ttaatagact ggatggaggc 1320
   ggataaagtt gcaggaccac ttctgcgctc ggcccttccg gctggctggt ttattgctga 1380
   taaatctgga gccggtgagc gtgggtctcg cggtatcatt gcagcactgg ggccagatgg 1440
   taagccctcc cgtatcgtag ttatctacac gacggggagt caggcaacta tggatgaacg 1500
   aaatagacag atcgctgaga taggtgcctc actgattaag cattggtaac tgtcagacca 1560
   agtttactca tatatacttt agattgattt aaaacttcat ttttaattta aaaggatcta 1620
   ggtgaagatc ctttttgata atctcatgac caaaatccct taacgtgagt tttcgttcca 1680
   ctgagcgtca gaccccgtag aaaagatcaa aggatcttct tgagatcctt tttttctgcg 1740
   cgtaatctgc tgcttgcaaa caaaaaaacc accgctacca gcggtggttt gtttgccgga 1800
   tcaagagcta ccaactcttt ttccgaaggt aactggcttc agcagagcgc agataccaaa 1860
   tactgtcctt ctagtgtagc cgtagttagg ccaccacttc aagaactctg tagcaccgcc 1920
   tacatacctc gctctgctaa tcctgttacc agtggctgct gccagtggcg ataagtcgtg 1980
   tcttaccggg ttggactcaa gacgatagtt accggataag gcgcagcggt cgggctgaac 2040
   ggggggttcg tgcacacagc ccagcttgga gcgaacgacc tacaccgaac tgagatacct 2100
   acagcgtgag cattgagaaa gcgccacgct tcccgaaggg agaaaggcgg acaggtatcc 2160
   ggtaagcggc agggtcggaa caggagagcg cacgagggag cttccagggg gaaacgcctg 2220
   gtatctttat agtcctgtcg ggtttcgcca cctctgactt gagcgtcgat ttttgtgatg 2280
   ctcgtcaggg gggcggagcc tatggaaaaa cgccagcaac gcggcctttt tacggttcct 2340
   ggccttttgc tggccttttg ctcacatgtt ctttcctgcg ttatcccctg attctgtgga 2400
   taaccgtatt accgcctttg agtgagctga taccgctcgc cgcagccgaa cgaccgagcg 2460
   cagcgagtca gtgagcgagg aagcggaaga gcgcctgatg cggtattttc tccttacgca 2520
   tctgtgcggt atttcacacc gcagaccagc cgcgtaacct ggcaaaatcg gttacggttg 2580
   agtaataaat ggatgccctg cgtaagcggg tgtgggcgga caataaagtc ttaaactgaa 2640
   caaaatagat ctaaactatg acaataaagt cttaaactag acagaatagt tgtaaactga 2700
   aatcagtcca gttatgctgt gaaaaagcat actggacttt tgttatggct aaagcaaact 2760
   cttcattttc tgaagtgcaa attgcccgtc gtattaaaga ggggcgtggc caagggcatg 2820
   gtaaagacta tattcgcggc gttgtgacaa tttaccgaac aactccgcgg ccgggaagcc 2880
   gatctcggct tgaacgaatt gttaggtggc ggtacttggg tcgatatcaa agtgcatcac 2940
   ttcttcccgt atgcccaact ttgtatagag agccactgcg ggatcgtcac cgtaatctgc 3000
   ttgcacgtag atcacataag caccaagcgc gttggcctca tgcttgagga gattgatgag 3060
   cgcggtggca atgccctgcc tccggtgctc gccggagact gcgagatcat agatatagat 3120
   ctcactacgc ggctgctcaa acctgggcag aacgtaagcc gcgagagcgc caacaaccgc 3180
   ttcttggtcg aaggcagcaa gcgcgatgaa tgtcttacta cggagcaagt tcccgaggta 3240
   atcggagtcc ggctgatgtt gggagtaggt ggctacgtct ccgaactcac gaccgaaaag 3300
   atcaagagca gcccgcatgg atttgacttg gtcagggccg agcctacatg tgcgaatgat 3360
   gcccatactt gagccaccta actttgtttt agggcgactg ccctgctgcg taacatcgtt 3420
   gctgctgcgt aacatcgttg ctgctccata acatcaaaca tcgacccacg gcgtaacgcg 3480
   cttgctgctt ggatgcccga ggcatagact gtacaaaaaa acagtcataa caagccatga 3540
   aaaccgccac tgcgccgtta ccaccgctgc gttcggtcaa ggttctggac cagttgcgtg 3600
   agcgcatacg ctacttgcat tacagtttac gaaccgaaca ggcttatgtc aactgggttc 3660
   gtgccttcat ccgtttccac ggtgtgcgtc acccggcaac cttgggcagc agcgaagtcg 3720
   aggcatttct gtcctggctg gcgaacgagc gcaaggtttc ggtctccacg catcgtcagg 3780
   cattggcggc cttgctgttc ttctacggca aggtgctgtg cacggatctg ccctggcttc 3840
   aggagatcgg tagacctcgg ccgtcgcggc gcttgccggt ggtgctgacc ccggatgaag 3900
   tggttcgcat cctcggtttt ctggaaggcg agcatcgttt gttcgcccag gactctagct 3960
   atagttctag tggttggcct acagctttgt ttaaacaaag ctgtacccgt agtggctatg 4020
   gcagggcttg ccgccccgac gttggctgcg agccctgggc cttcacccga acttgggggt 4080
   tggggtgggg aaaaggaaga aacgcgggcg tattggtccc aatggggtct cggtggggta 4140
   tcgacagagt gccagccctg ggaccgaacc ccgcgtttat gaacaaacga cccaacaccc 4200
   gtgcgtttta ttctgtcttt ttattgccgt catagcgcgg gttccttccg gtattgtctc 4260
   cttccgtgtt tcagttagcc tcccccatct cccggtaccg catgctatgc atcagctgct 4320
   agcaccatgg ctcgagatcc cgggtgatca agtcttcgtc gagtgattgt aaataaaatg 4380
   taatttacag tatagtattt taattaatat acaaatgatt tgataataat tcttatttaa 4440
   ctataatata ttgtgttggg ttgaattaaa ggtccgtata ctagtatcga ttcgcgacct 4500
   actccggaat attaatagat catggagata attaaaatga taaccatctc gcaaataaat 4560
   aagtatttta ctgttttcgt aacagttttg taataaaaaa acctataaat attccggatt 4620
   attcataccg tcccaccatc gggcgcggat cccggtccga agcgcgcgga attcaaaggc 4680
   ctacgtcgac gagctcacta gtcgcggccg ctttcgaatc tagagcctgc agtctcgaca 4740
   agcttgtcga gaagtactag aggatcataa tcagccatac cacatttgta gaggttttac 4800
   ttgctttaaa aaacctccca cacctccccc tgaacctgaa acataaaatg aatgcaattg 4860
   ttgttgttaa cttgtttatt gcagcttata atggttacaa ataaagcaat agcatcacaa 4920
   atttcacaaa taaagcattt ttttcactgc attctagttg tggtttgtcc aaactcatca 4980
   atgtatctta tcatgtctgg atctgatcac tgcttgagcc taggagatcc gaaccagata 5040
   agtgaaatct agttccaaac tattttgtca tttttaattt tcgtattagc ttacgacgct 5100
   acacccagtt cccatctatt ttgtcactct tccctaaata atccttaaaa actccatttc 5160
   cacccctccc agttcccaac tattttgtcc gcccacagcg ggcatttttt cttcctgtta 5220
   tgtttttaat caaacatcct gccaactcca tgtgacaaac cgtcatcttc ggctacttt 5279
<210> 2
   <211> 3005
   <212> DNA
   <213> baculovirus
<220>
   <221> misc_feature
   <223> Description of sequence: baculovirus vector pUCDM
<400> 2
   aattctgtca gccgttaagt gttcctgtgt cactgaaaat tgctttgaga ggctctaagg      60
   gcttctcagt gcgttacatc cctggcttgt tgtccacaac cgttaaacct taaaagcttt     120
   aaaagcctta tatattcttt tttttcttat aaaacttaaa accttagagg ctatttaagt     180
   tgctgattta tattaatttt atggtcaaac agagagctta gtacgtgaaa catgagagct     240
   tagtacgtta gccatgagag cttagtacgt tagccatgag ggtttagttc gttaaacatg     300
   agagcttagt acgttaaaca tgagagctta gtacgtgaaa catgagagct tagtacgtac     360
   tatcaacagg ttgaactgct gatcaacaga tcctctacgc ggccgcggta ccataacttc     420
   gtatagcata cattatacga agttatctgg tttaaacgta cccgtagtgg ctatggcagg     480
   gcttgccgcc ccgacgttgg ctgcgagccc tgggccttca cccgaacttg ggggttgggg     540
   tggggaaaag gaagaaacgc gggcgtattg gtcccaatgg ggtctcggtg gggtatcgac     600
   agagtgccag ccctgggacc gaaccccgcg tttatgaaca aacgacccaa cacccgtgcg     660
   ttttattctg tctttttatt gccgtcatag cgcgggttcc ttccggtatt gtctccttcc     720
   gtgtttcagt tagcctcccc catctcccgg taccgcatgc tatgcatcag ctgctagcac     780
   catggctcga gatcccgggt gatcaagtct tcgtcgagtg attgtaaata aaatgtaatt     840
   tacagtatag tattttaatt aatatacaaa tgatttgata ataattctta tttaactata     900
   atatattgtg ttgggttgaa ttaaaggtcc gtatactagt atcgattcgc gacctactcc     960
   ggaatattaa tagatcatgg agataattaa aatgataacc atctcgcaaa taaataagta    1020
   ttttactgtt ttcgtaacag ttttgtaata aaaaaaccta taaatattcc ggattattca    1080
   taccgtccca ccatcgggcg cggatcccgg tccgaagcgc gcggaattca aaggcctacg    1140
   tcgacgagct cactagtcgc ggccgctttc gaatctagag cctgcagtct cgacaagctt    1200
   gtcgagaagt actagaggat cataatcagc cataccacat ttgtagaggt tttacttgct    1260
   ttaaaaaacc tcccacacct ccccctgaac ctgaaacata aaatgaatgc aattgttgtt    1320
   gttaacttgt ttattgcagc ttataatggt tacaaataaa gcaatagcat cacaaatttc    1380
   acaaataaag catttttttc actgcattct agttgtggtt tgtccaaact catcaatgta    1440
   tcttatcatg tctggatctg atcactgctt gagcctagaa gatccggctg ctaacaaagc    1500
   ccgaaaggaa gctgagttgg ctgctgccac cgctgagcaa taactagcat aaccccttgg    1560
   ggcctctaaa cgggtcaacc cctaggggcc tctaaacggg tcttgagggg ttttttgctg    1620
   aaaggaggaa ctatatccgg atctgaacag gagggacagc tgatagaaac agaagccact    1680
   ggagcacctc aaaaacacca tcatacacta aatcagtaag ttggcagcat cacccgacgc 1740
   actttgcgcc gaataaatac ctgtgacgga agatcacttc gcagaataaa taaatcctgg 1800
   tgtccctgtt gataccggga agccctgggc caacttttgg cgaaaatgag acgttgatcg 1860
   gcacgtaaga ggttccaact ttcaccataa tgaaataaga tcactaccgg gcgtattttt 1920
   tgagttatcg agattttcag gagctaagga agctaaaatg gagaaaaaaa tcactggata 1980
   taccaccgtt gatatatccc aatggcatcg taaagaacat tttgaggcat ttcagtcagt 2040
   tgctcaatgt acctataacc agaccgttca gctggatatt acggcctttt taaagaccgt 2100
   aaagaaaaat aagcacaagt tttatccggc ctttattcac attcttgccc gcctgatgaa 2160
   tgctcatccg gaattccgta tggcaatgaa agacggtgag ctggtgatat gggatagtgt 2220
   tcacccttgt tacaccgttt tccatgagca aactgaaacg ttttcatcgc tctggagtga 2280
   ataccacgac gatttccggc agtttctaca catatattcg caagatgtgg cgtgttacgg 2340
   tgaaaacctg gcctatttcc ctaaagggtt tattgagaat atgtttttcg tctcagccaa 2400
   tccctgggtg agtttcacca gttttgattt aaacgtggcc aatatggaca acttcttcgc 2460
   ccccgttttc accatgggca aatattatac gcaaggcgac aaggtgctga tgccgctggc 2520
   gattcaggtt catcatgccg tctgtgatgg cttccatgtc ggcagaatgc ttaatgaatt 2580
   acaacagtac tgcgatgagt ggcagggcgg ggcgtaattt ttttaaggca gttattggtg 2640
   cccttaaacg cctggtgcta cgcctgaata agtgataata agcggatgaa tggcagaaat 2700
   tcgaaagcaa attcgacccg gtcgtcggtt cagggcaggg tcgttaaata gccgcttatg 2760
   tctattgctg gtttaccggt ttattgacta ccggaagcag tgtgaccgtg tgcttctcaa 2820
   atgcctgagg ccagtttgct caggctctcc ccgtggaggt aataattgac gatatgatca 2880
   tttattctgc ctcccagagc ctgacattca tccggggtca gcaccgtttc tgcggactgg 2940
   ctttctacgt gttccgcttc ctttagcagc ccttgcgccc tgagtgcttg cggcagcgtg 3000
   aagct 3005